# EUROPEAN PATENT SPECIFICATION

(11) **EP 0 420 116 B1**
(45) Date of publication and mention of the grant of the patent: **30.07.1997**
(21) Application number: 90118331.9
(22) Date of filing: 24.09.1990
(51) Int. Cl.: C07D 295/14, C07D 295/06, A61K 31/445

(54) **Novel substituted alkyl piperidines and their use as inhibitors of cholesterol synthesis**
Substituierte Alkylpiperidine und deren Verwendung als Inhibitoren der Cholesterolsynthese
Alkylpipéridines substitués et leur utilisation comme inhibiteurs de la synthèse du cholestérol

(30) Priority: 22.09.1989 US 412037; 27.07.1990 US 557091
(43) Date of publication of application: 03.04.1991
(73) Proprietor: MERRELL PHARMACEUTICALS INC., Cincinnati, Ohio 45215-6300 (US)
(72) Inventor: McCarthy, James Ray, West Chester, Ohio 45069 (US); Wannamaker, Marion Woods, Cincinnati, Ohio 45215 (US); Barney, Charlotte Louise, Cincinnati, Ohio 45212 (US)
(74) Representative: VOSSIUS & PARTNER

(56) References cited:
- EP-A- 029 618
- EP-A- 0 235 942
- CH-A- 505 251
- DE-A- 3 011 504
- DE-B- 1 145 616
- DE-C- 866 193
- FR-A- 1 261 160
- FR-A- 2 272 643
- GB-A- 1 415 682
- GB-A- 1 420 758
- US-A- 2 580 411
- US-A- 3 185 678
- US-A- 4 326 067
- SYNTHESIS, 1983, N 9, p. 723-725
- J. CHEM. SOC., Perkin.II, 1984, p. 349-354
- CHEM. ABSTR., Vol.84, no. 105360e
- CHEMICAL ABSTRACTS, Vol. 104, no. 6, 10th February 1986, page 612, abstract no. 43107y.
- CHEMICAL ABSTRACTS, vol. 51, no. 17, 10th September 1957, abstract no. 12097g; A.P. TERENT'EV et al.: "Synthesis with acrylonitrile. XXX. Some 1-substituted pyrrolidines and piperidines"
- TETRAHEDRON LETTERS, vol. 31, no. 39, 17th September 1990, pages 5547-5550; C.L. BARNEY et al.: "A convenient synthesis of hindered amines and a-trifluoromethylamines from ketones"

## Description

The present invention relates to a group of compounds which are novel substituted alkyl piperidines and which act to inhibit the synthesis of cholesterol in mammals and in fungi.

Vascular disease, because of its effects upon the brain, heart, kidneys, extremities, and other vital organs, is a leading cause of morbidity and mortality in the United States and in most Western countries. In this regard, much has been learned about arteriosclerosis, atherosclerosis, and the lipidemias, with particular reference to cholesterol. In particular, there is convincing evidence of a reciprocal relationship between a high serum cholesterol and the incidence of atherosclerosis and its complications. Much interest has been expressed in recent years in reducing the level of serum cholesterol. However, some studies have shown that even radical reductions in dietary cholesterol achieves only a modest decrease of 10 to 15% in plasma cholesterol. Thus, it has been appreciated that further reductions in serum cholesterol will require other therapeutic measures, including the physiological inhibition of cholesterol synthesis in the body.

The enzymatic biosynthesis of cholesterol is a complex process, which requires altogether some 25 reaction steps. The pathway can be divided into three stages: (1) the conversion of acetic acid to mevalonic acid; (2) the conversion of mevalonic acid into squalene; and (3) the conversion of squalene into cholesterol. In the last stage of cholesterol biosynthesis, squalene is converted to squalene 2,3-epoxide via oxidation, a reaction catalyzed by squalene monooxygenase, also known as squalene epoxidase. The squalene 2,3-epoxide then undergoes cyclization to lanosterol, the first sterol to be formed.

The cyclization of 2,3-oxidosqualene to lanosterol is a key reaction in the biosynthesis of cholesterol in animals. The reaction is catalyzed by the microsomal enzyme 2,3-oxidosqualene lanosterol-cyclase. (See generally, Taylor, Frederick R., Kandutsch, Andrew A., Gayen, Apurba K., Nelson, James A., Nelson, Sharon S., Phirwa, Seloka, and Spencer, Thomas A., *24,25-Epoxysterol Metabolism in Cultured Mammalian Cells and Repression of 3-Hydroxy-3-methylglutaryl-CoA Reductase*, The Journal of Biological Chemistry, 261, 15039-15044 (1986), incorporated herein by reference.)

In addition, it has recently been reported that certain compounds, such as allylamines, act as potent inhibitors of fungal squalene epoxidase. Fungal infections (mycoses) are found throughout the world. Only a few structural classes of compounds currently satisfy the demands of modern chemotherapy in their treatment and the search for new types of active substances is of major therapeutic importance. (See generally, Stutz, Anton, *Allylamine Derivatives-A New Class of Active Substances in Antifungal Chemotherapy,* Angew. Chem. Int. Ed. Engl., 26, 320-328 (1987).) As inhibitors of squalene epoxidase in animals, the compounds of the present invention are believed to be useful in the treatment of fungal infections through the inhibition of cholesterol synthesis.

Chemical Abstracts, Vol. 51, No. 12097g, DE-C-866193, DE-A 3 011 504, GB-A-1 420 758, GB-A 1 415 682, DE-B-1 145 616, FR-A-1 261 160, US-A-3 185 678 and EP-A-0 235 942 describe derivatives of piperidine and related compounds, their preparation and in some cases the use of the compounds in pharmaceutical compositions, e.g. as anti-ulcer or antibacterial agents.

The present invention relates to compounds having the following general formula: 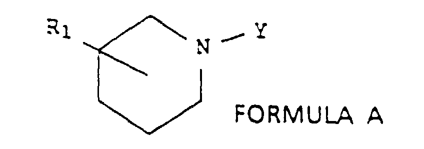 and the pharmaceutically acceptable salts thereof wherein Y is -A-(Alk₁)ₓ-D-(Alk₂)-E-(Alk₃)_{y}-CH₃ wherein, the moiety A is -CH₂- or 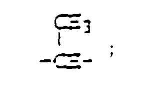 D and E are each independently 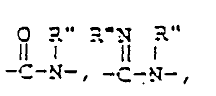 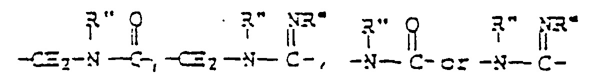 or a direct bond, with the proviso that when D is a moiety from the group 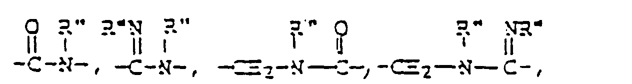 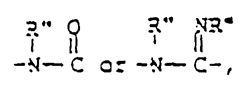 E cannot be a moiety from the same group, and that D and E cannot both be direct bonds;
- (Alk₁), (Alk₂) and (Alk₃): are each independently a straight chain alkylene moiety containing up to 5 carbon atoms, optionally substituted with up to 3 methyl groups, or,
- (Alk₁), (Alk₂) and (Alk₃): are each independently a straight chain alkenylene moiety containing from 2 to 6 carbon atoms, the straight chain alkenylene moiety having 1 to 2 double bonds, and optionally substituted with up to 3 methyl groups;
- R₁: is hydrogen, hydroxy or C₁₋₄ lower alkyl;
- R', R" and R"': are each independently hydrogen or or C₁₋₄ lower alkylₓ; and x and y independently represent 0 or 1; with the proviso that when A is -CH₂- or -CH₇(CH₃)- and one of D or E is a direct bond, the other of D and E is not -C(O)N(R")- except when the compound of formula (A) is N-(3-methylbutyl)1-piperidine propanamide, N-butyl-N-methyl-1-piperidinebutyramide or 4-(3-methylbutylamino)-1-piperidinobutane and the pharmaceutically acceptable salts thereof.

As used in this application:
(a) the term alkylene refers to methylene, ethylene, propylene, butylene, pentylene and hexylene;
(b) the term alkenylene refers to any of the above alkylenes having 1 or 2 double bonds along the chain thereof; and,
(c) the term C₁₋₄ lower alkyl refers to methyl, ethyl, propyl, isopropyl, butyl, sec-butyl and tert-butyl.

Also, as used in this application, the substituent represented as R₁ may be at any position from 2-6 around the piperidine ring. There may be up to three such independent substitutions around the piperidine ring wherein the substituent is other than hydrogen.

In general, compounds of the present invention are prepared by the following methods.

### REACTION SCHEME I

Compounds according to Formula A wherein D is 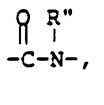 the moiety A is -CH₂- or 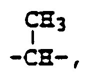 E is a direct bond, y is 0 and (Alk₁), (Alk₂), R" and X are defined as in Formula A, can be made according to the following reaction scheme, to form a product according to Formula I.

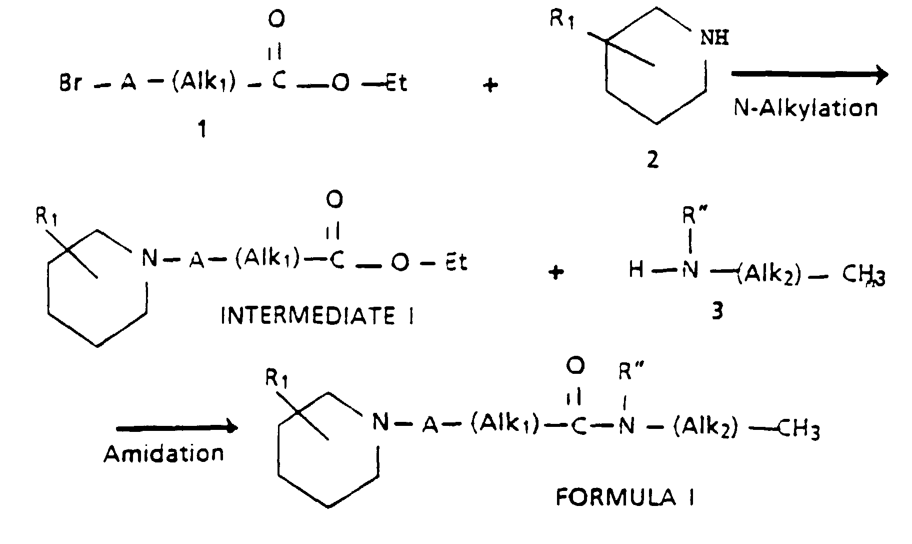

The first step in the reaction is the N-alkylation of piperidine by a bromoalkyl ester compound represented by structure 1. It will be understood that the abbreviation Br in this example and as used throughout this application represents bromine; however, the chloroalkyl ester may also be utilized. Likewise, it will be understood that the abbreviation Et in this example and as used throughout this application represents ethyl; however, other alkyl esters such as the methyl, propyl or isopropyl esters, for example, may also be utilized. The piperidine can be optionally substituted by R₁, as defined above and as represented by structure 2.

The appropriate starting compounds are a bromoalkyl ester wherein A and Alk₁ have the same definitions as that desired in the final product and a piperidine wherein R₁ has the same definition as desired in the final product, as represented by Formula I.

The alkylation reaction can be performed by techniques well known in the art. Typically the bromoalkyl ester 1 and the piperidine 2 are mixed in approximately a 1:2 molar ratio in a solvent, such as benzene, and the reaction mixture is heated at reflux under a nitrogen atmosphere, for example, for approximately 16 hours. Intermediate I is then recovered from the reaction mixture and purified by techniques known in the art. For example, the reaction mixture is concentrated under reduced pressure and then taken up in ether and filtered to give Intermediate I as shown.

The next step in the reaction scheme is an amidation reaction between intermediate I and a substituted amine as shown in structure 3. The substituted amine chosen is one in which R" and Alk₂ have the same definition as R" and Alk₂ in the final product, represented by Formula I. The substituted amine and Intermediate I are contacted in approximately a 2:1 molar ratio in the presence of 2-hydroxypyridine and heated at approximately 60°C for approximately 72 hours. The solution is poured into water, extracted with ethyl acetate, dried over magnesium sulfate and concentrated under reduced pressure. The final product is further purified using chromatographic techniques well known in the art such as flash chromatography.

### REACTION SCHEME III

Alternatively, the amide according to Formula I, as prepared and defined above, can be converted to the corresponding amidine, which is a representation of Formula A, wherein D is 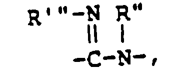 by first reacting the amide of Formula I with triethyloxonium tetrafluoroborate followed by addition of an amine of the structure R'"-NH₂ to form an amidine according to Formula III. 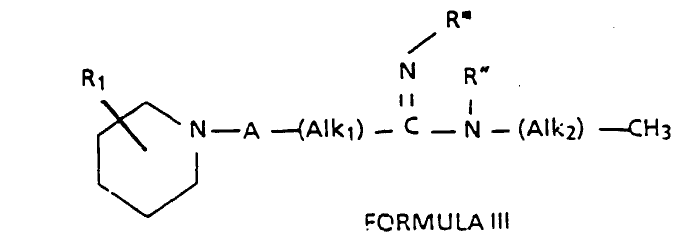 The amine is chosen such that R"' has the same definition as the definition of R"' desired in the final product, as represented by Formula III.

An amide prepared according Reaction Scheme I is dissolved in methylene chloride and treated with approximately an equimolar amount of triethyloxonium tetrafluoroborate followed by an excess amount of the desired substituted amine. The reaction mixture is stirred overnight at room temperature followed by heating the reaction mixture to reflux.

The solution is then cooled to room temperature, quenched with water, dried over a drying agent such as magnesium sulfate and concentrated under reduced pressure. The resulting oil can be purified by techniques well known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula III.

### REACTION SCHEME IV

Amidines according to Formula IV can be prepared by the following reaction scheme. Formula IV is a representation of Formula A, wherein D is 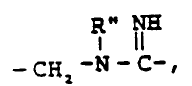 the moiety A is -CH₂-, or 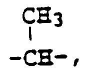 R"' is hydrogen, Y is 0, E is a direct bond and (Alk₁), (Alk₂), R₁, R" and X are defined as in Formula A.

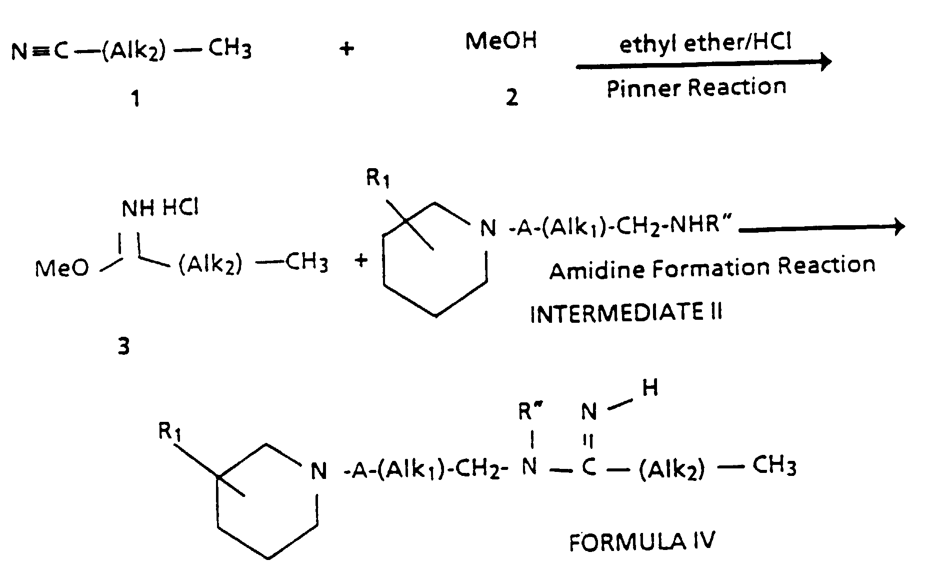

The first step in the reaction sequence is to conduct a Pinner reaction converting alkylnitrile 1, wherein (Alk₂) has the same definition as that desired in the final product, to the imidate ester 3. The alkylnitrile 1 and an appropriate alcohol such as methanol 2 are mixed in approximately equimolar amounts in a solvent, such as ethyl ether and cooled to 0°C, then saturated with hydrochloric acid and stirred overnight at room temperature. The imidate ester 3 is concentrated under reduced pressure and can be purified by standard techniques such as trituration in ether, filtration and air drying. The resulting salt is taken up in ether, treated with cold saturated sodium bicarbonate, the layers separated and the aqueous layer washed with ether. The combined organics are dried, filtered and concentrated under reduced pressure to give the purified intermediate product 3.

The imidate ester 3 is then mixed with an appropriate N-substituted alkylamine piperidine represented by Intermediate II in a amidine formation reaction.

Intermediate II A (intermediate II, wherein R" is hydrogen) is obtainable as follows:

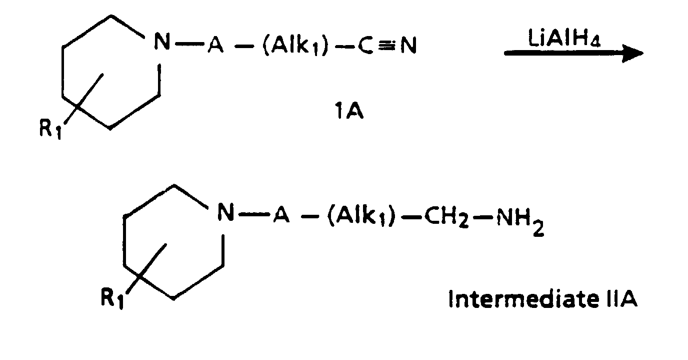

The alkylcyano piperidine 1A is chosen such that A and (Alk₁) have the same definition as that desired in the final product. The alkylcyano piperidine 1A is mixed, dropwise, with an approximately equimolar amount of lithium aluminum hydride (LiAlH₄) powder in tetrahydrofuran. The reaction mixture is kept at room temperature overnight, quenched with water and sodium hydroxide, filtered, washed and dried over magnesium sulfate, and concentrated under reduced pressure to give Intermediate II.

Intermediate II B (Intermediate II, wherein R" is C₁-C₄ lower alkyl) is obtainable as follows:

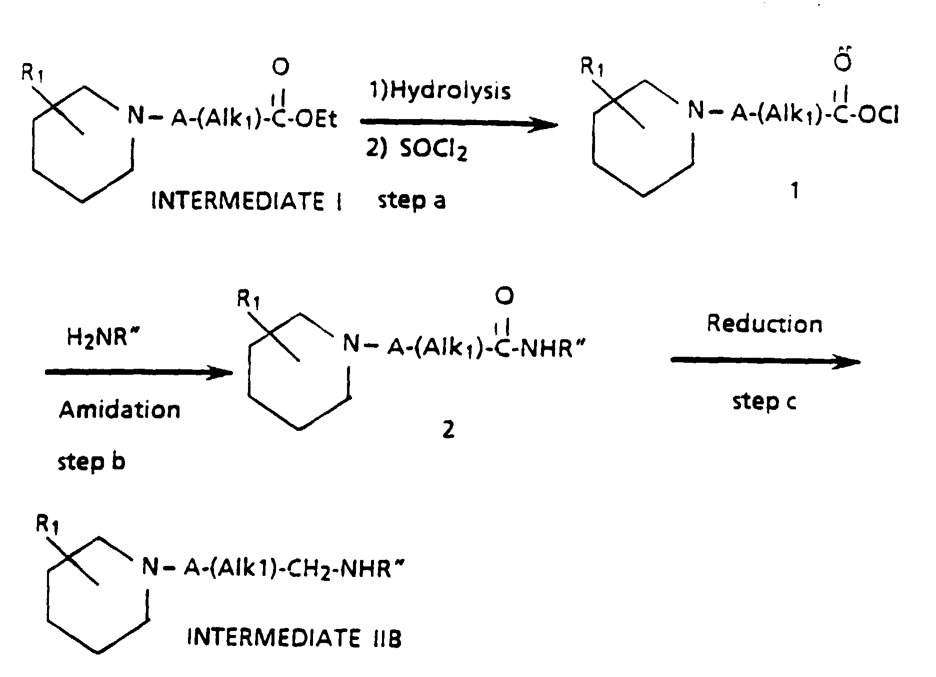

Intermediate II and the imidate ester 3 are mixed in approximately equimolar amounts and allowed to stand overnight at room temperature.

The final product according to Formula IV can be purified by standard techniques, i.e. the resulting oil is taken up in ether, filtered or treated with anhydrous hydrochloric acid, treated with sodium hydroxide, extracted with ether, dried over magnesium sulfate and concentrated under reduced pressure. The resulting oil can be dissolved in ether, filtered and treated with anhydrous hydrochloric acid. The resulting precipitate is filtered and recrystallized in ethyl acetate/isopropyl alcohol, for example, to give the final product according to Formula IV.

### REACTION SCHEME V

The above amidine according to Formula IV, as prepared and defined above, can be hydrolyzed by treatment with hydrochloric acid to form an amide according to Formula V, which is a representation of Formula A, wherein D is 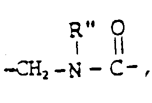 E is a direct bond and Y is 0. 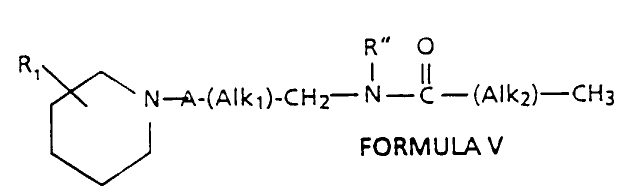 The final product of Formula V as its hydrochloric acid salt can be collected by filtration and air dried.

### REACTION SCHEME VI

The amide of Formula V, as prepared and defined above, can be treated with an alkylating agent, such as triethyloxonium tetrafluoroborate, followed by introduction of an excess amount of a substituted amine H₂NR'" to form a substituted amidine according to Formula VI, wherein D is 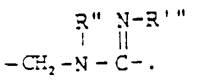 The amine H₂NR"' is the chosen so that R"' has the same definition as that desired in the final product. 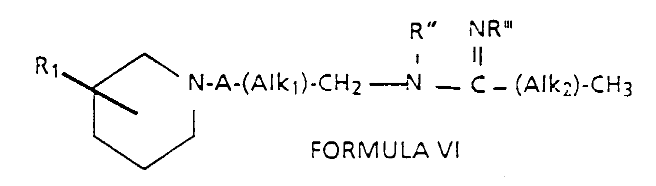 The final product according to Formula VI can be purified according to techniques well-known in the art. The resulting oil can be dissolved in ether, filtered and treated with anhydrous hydrochloric acid. The resulting precipitate is filtered and recrystallized in ethyl acetate/isopropyl alcohol, for example, to give the final product according to Formula VI.

In addition, complex compounds in which the alkyl side chain can include both an amine and an amide, or an amine and an amidine or an amide and an amidine can be prepared by the following reaction schemes.

### REACTION SCHEME IX

Compounds according to following Formula IX, which is a representation of Formula A, wherein the moiety A is -CH₂- or 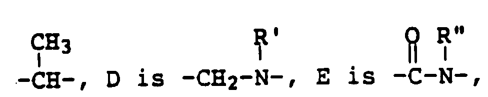 and (Alk₁), (Alk₂), (Alk₃), R', R" and R₁ are defined as in Formula A, can be prepared by the following method.

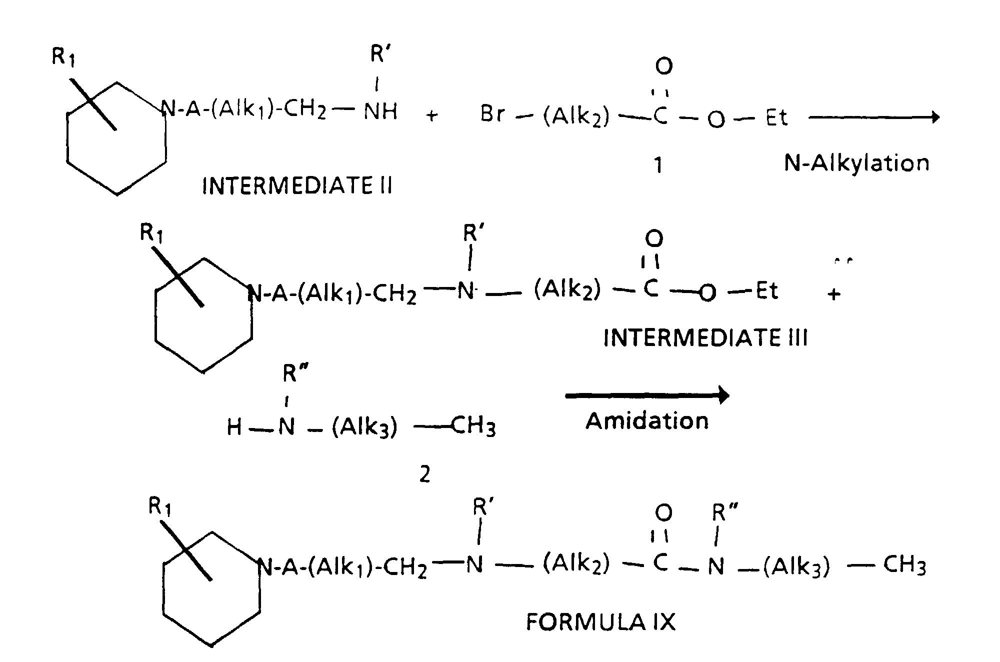

The first step in Reaction Scheme IX is the N-alkylation of the N-substituted piperidine represented by Intermediate II (from Reaction Scheme IV) by the bromoalkyl ester 1. The appropriate starting materials are a bromoalkyl ester in which (Alk₂) has the same definition as that desired in the final product and a substituted piperidine in which R', A and (Alk₁) have the same definitions as desired in the final product.

The alkylation reaction can be conducted utilizing techniques well known in the art. Typically the bromoalkyl ester 1 and the substituted piperidine represented by Intermediate II are mixed in approximately a 1:2 molar ratio in a solvent, such as benzene, and the reaction mixture is heated at reflux under a nitrogen atmosphere, for example, for approximately 16 hours. Intermediate III is then recovered from the reaction mixture and purified by techniques known in the art. For example, the reaction mixture is concentrated under reduced pressure and then taken up in ether and filtered to give the Intermediate III as shown.

The next step in the reaction scheme is an amidation reaction between Intermediate III and a substituted amine as shown in structure 2. The substituted amine chosen is one in which R" and Alk₃ have the same definition as R" and Alk₃ in the final product, represented by Formula IX. The substituted amine and Intermediate III are contacted in approximately a 2:1 molar ratio in the presence of 2-hydroxypyridine and heated at 60°C for approximately 72 hours. The solution is poured into water, extracted with ethyl acetate, dried over magnesium sulfate and concentrated under reduced pressure. The final product is further purified using chromatographic techniques well known in the art such as flash chromatography.

### REACTION SCHEME X

A compound according to Formula IX, as prepared and defined above, can be converted to the corresponding amidine by reacting it with triethyloxonium tetrafluoroborate, followed by an amine of the structure R"'-NH₂ to form a compound according to the following Formula X, which is a representation of Formula A wherein E is 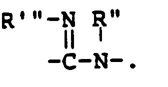 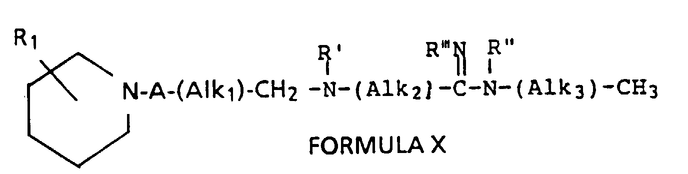 The amine is chosen so that R'" has the same definition as the definition of R'" desired in the final product. An amide prepared according Reaction Scheme IX is dissolved in methylene chloride and treated with an approximately equimolar amount of triethyloxonium tetrafluoroborate, followed by an excess amount of the desired substituted amine. The final product according to Formula X can be described in further detail in Reaction Scheme IX above.

As examples of compounds of the present invention are the following:
1. N-(3-Methylbutyl)-1-piperidine propanamide hydrochloride.
2. 4-(3-Methylbutylamino)-1-piperidinobutane dihydrochloride.
3. N-(3-Piperidinopropyl)-4-methylvalerylamidine.
4. N-Butyl-N-methyl-1-piperidinebutyramide.
5. N-(4-Piperidinobutyl)-4-methylvalerylamidine.
6. Piperidine octylamidine hydrochloride.
7. N-(1-trifluoromethyl-undecane)-piperidine.

The following assays are used to test compounds for their ability to inhibit 2,3-oxidosqualene lanosterol-cyclase or epoxidase. Microsomes, prepared by ultracentrifugation of homogenates of rat liver, are incubated at 37°C for 45 minutes in the presence of 60 µM 3H-squalene, 2.0 mM NADPH, 0.01 mM FAD, and the high speed supernatant fraction from the microsomal preparation. Blanks, in which NADPH has been omitted, are run simultaneously with the test compounds. Compounds are tested at concentrations of >0.0 to 100.0 µM.

### Method 1

Following incubation the samples are saponified, standards are added to each sample, and then the reaction products are extracted into hexane. The hexane extracts are dried and then the dried extracts are redissolved in chloroform. The reaction products contained in the extracts are then separated by thin layer chromatography (TLC). Spots containing the reaction products are scraped from the TLC plates and counted for radioactivity in a scintillation counter. An IC₅₀ is finally calculated.

### Method 2

Following incubation reactions are stopped by the addition of chloroform:methanol, standards are added, then reaction products and standards are extracted into chloroform. The chloroform extracts are dried, and the residue is dissolved in toluene:methanol. The reaction products and standards contained in the dissolved residue are separated by high performance liquid chromatography (HPLC). Chromatographic peaks containing reaction products are monitored for radioactivity with a flow-through scintillation counter connected in series with the HPLC column. An IC₅₀ is calculated based on the radioactivity in controls and samples.

### Pharmaceutical Preparations of the 2,3-Oxidosqualene Lanosterol-Cyclase Inhibitors

The compounds of this invention are useful both in the free base form and in the form of acid addition salts. The acid addition salts are simply a more convenient form for use and, in practice, use of the salt amounts to use of the free base. The expression "pharmaceutically acceptable acid addition salts" is intended to apply to any non-toxic organic or inorganic acid addition salts of the base compounds of the above compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the sulfonic acids such as p-toluenesulfonic, methanesulfonic acid and 2-hydroxyethanesulfonic acid. Either the mono- or the di-acid salts can be formed, and such salts can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution.

The preferred route of administration is oral administration. For oral administration the compounds can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

The compounds of this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethyleneglycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures.

The parenteral compositions of this invention will typically contain from about 0.5 to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17 % by weight. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The exact amount of the compound or compounds to be employed, i.e., the amount of the subject compound or compounds sufficient to provide the desired effect, depends on various factors such as the compound employed; type of administration; the size, age and species of animal; the route, time and frequency of administration; and, the physiological effect desired. In particular cases, the amount to be administered can be ascertained by conventional range finding techniques.

The compounds are preferably administered in the form of a composition comprising the compound in admixture with a pharmaceutically acceptable carrier, i.e., a carrier which is chemically inert to the active compound and which has no detrimental side effects or toxicity under the conditions of use. Such compositions can contain from about 0.1 µg or less to 500 mg of the active compound per ml of carrier to about 99% by weight of the active compound in combination with a pharmaceutically-acceptable carrier.

The compounds may also be incorporated into any inert carrier so that they may be utilized in routine serum assays, blood levels, urine levels, etc., according to techniques well known in the art.

The compositions can be in solid forms, such as tablets, capsules, granulations, feed mixes, feed supplements and concentrates, powders, granules or the like; as well as liquid forms such as sterile injectable suspensions, orally administered suspensions or solutions. The pharmaceutically acceptable carriers can include excipients such as surface active dispersing agents, suspending agents, tableting binders, lubricants, flavors and colorants. Suitable excipients are disclosed, for example, in texts such as *Remington's Pharmaceutical Manufacturing,* 13 Ed., Mack Publishing Co., Easton, Pennsylvania (1965).

The following examples are presented to illustrated the present invention but they should not be construed as limiting in any way.

### EXAMPLE 1

Ethyl 4-bromobutyroate (10.0 g, 0.0513 mmol) and 9.37 g (0.110 mmol) of piperidine were mixed in benzene (25 ml) and heated at reflux under nitrogen atmosphere overnight.

The reaction mixture was concentrated under reduced pressure, taken up into ether and filtered. The filtrate was shaken with saturated sodium bicarbonate, the layers separated, the aqueous layer extracted with ethyl ether (2 x 50 ml). The combined organics were dried over magnesium sulfate and concentrated under reduced pressure to give 8.68 g of a yellow oil (85%).

Next, 4.0 g (0.02 mmol) of the above product was mixed with 4.4 g (0.05 mmol) isopentylamine in the presence of 1.4 g (0.015 mmol) 2-hydroxypyridine and heated together at 60°C in a closed tube (screw cap) and followed by gas chromatography. After 72 hours, the solution was poured into 200 ml water, extracted with ethyl acetate (3 x 75 ml), dried over magnesium sulfate and concentrated under reduced pressure to give 4.21 g of an orange oil (50% yield). Flash chromatography (methylene chloride followed by 10% methanol/methylene chloride) gave 3.1 g of an orange oil.

The above product (2.08 mmol) was dissolved in ether and treated with gaseous hydrochloric acid to give a pale orange solid. Recrystallization (ethyl acetate/isopropyl alcohol) gave 296 mg of a white crystal product N-(3-methylbutyl)-1-piperidine propanamide hydrochloride (m.p. 77-82°C). Anal. Calcd. for C₁₄H₂₈N₂O HCl 1.3 H₂O: C, 56.00; H, 10.61; N, 9.53; Found: C,56.17; H, 10.28; N, 9.36.

### EXAMPLE 2

The above product (1.0 g, 0.00416 mmol) was dissolved in tetrahydrofuran (25 ml) in an oven-dried, 3-necked, 100 ml round-bottomed flask, equipped with a stir bar, thermometer, N₂-line and addition funnel (rubber septum). This was cooled to 10°C and 5 ml (1.0M in ethyl ether, 0.005 mmol) lithium aluminum hydride (LAH) added dropwise. The reaction mixture was stirred overnight at room temperature then heated at reflux. An additional 5 ml LAH solution was added and the reaction continued at reflux. The solution was cooled to room temperature, quenched with 400 ml of water, dried over magnesium sulfate and concentrated under reduced pressure to give 740 mg of a low melting, oily solid (79%). The oil was dissolved in ether, filtered and treated with anhydrous hydrochloric acid. The resulting precipitate was filtered, recrystallized (ethyl acetate/isopropyl alcohol) to give a white solid, m.p. 261-264°C 4-(3-methylbutylamino)-1-piperidinobutane dihydrochloride. Anal. Calcd. for C₁₄,H₃₀N₂ 2HCl H₂O: C, 52.99,; H, 10.80; N, 8.83. Found: C, 53.34; H, 10.82; N, 8.93.

### EXAMPLE 3

4-Methylvaleronitrile (10.0 g, 0.103 mmol) and 100 ml of ethyl ether were mixed in 3.5 g (0.110 mmol) methanol and cooled to 0°C, then saturated with hydrochloric acid and stirred overnight at room temperature.

The reaction mixture, which contained a small amount of white precipitate, was concentrated under reduced pressure, triturated with ether, filtered and air dried to give 10.5 g of a white fluffy solid, m.p. 105-105.5°C.

Next, 1.0 g of the above hydrochloric acid salt was taken up in ether, treated with cold saturated sodium bicarbonate, the layers separated, the aqueous layer washed with ether (2 x 10 ml), the combined organics dried, filtered and concentrated under reduced pressure to give 470 mg of a clear oil (60.3%).

The above product (9.5 g, 0.0573 mmol) was mixed with 8.16 g (0.0573 mmol) of 3-piperidino-1-propylamine and allowed to stand overnight at room temperature (CaCl₂ tube). The reaction mixture was concentrated under reduced pressure to give 16.5 g of an orange oil. The oil was redissolved in 50 ml methanol, treated with decolorizing carbon, filtered and concentrated under reduced pressure to give 15.4 g of a yellow oil. The oil was treated with saturated sodium bicarbonate, then 5N sodium hydroxide, extracted 3 times with ethyl acetate, dried over magnesium sulfate/potassium carbonate and concentrated under reduced pressure to give 6.0 g of a clear oil, which was taken up in anhydrous ether, filtered and treated with anhydrous hydrochloric acid. It was then treated with 5N sodium hydroxide, extracted with ether (3 x 50 ml), dried over magnesium sulfate/potassium carbonate and concentrated under reduced pressure to give 4.8 g of N-(3-piperidinopropyl)-4-methylvalerylamidine as a clear oil.

### EXAMPLE 4

First, 10.0 g (0.05 mmol) of ethyl 4-bromobutyrate and 9.37 g (0.110 mmol) of piperidine were mixed in 50 ml of benzene in a 100 ml, 1-necked, round-bottomed flask and heated at reflux under N₂-atmosphere overnight.

The reaction mixture, which contained a white precipitate, was treated with saturated sodium bicarbonate, the layers separated, the aqueous layer washed with ethyl acetate (2 x 30 ml), the combined organics dried over magnesium sulfate and concentrated under reduced pressure to give 8.57 g of a yellow oil (83.8%).

Next, 2.4 ml (20 mmol) of N-methylbutylamine and 50 ml of anhydrous methylene chloride were placed in an oven-dried, 3-necked 100 ml round-bottomed flask equipped with a stir bar, thermometer, addition funnel (rubber septum), and N₂-line. Trimethylaluminum (10 ml, 20 mmol, 2.0M in toluene) was added dropwise at room temperature and the reaction mixture stirred for 15 min. The above oil (4.0 g, 20 mmol) in 15 ml anhydrous methylene chloride was added dropwise and the reaction monitored by gas chromatography.

The reaction mixture was carefully quenched with 1N HCl, basified with 5N sodium hydroxide, the layers separated, the aqueous extracted with methylene chloride (2 x 50 ml), dried over magnesium sulfate and concentrated under reduced pressure to give 4.12 g of product (86% yield). Flash chromatography (methylene chloride, then 10% methanol/methylene chloride) gave 1.95 g of a yellow, low melting solid, N-butyl-N-methyl-1-piperidinebutyramide.

### EXAMPLE 5

Lithium aluminum hydride (2.5 g, 0.068 mmol, powder) was placed in an oven-dried, 3-necked, round-bottomed flask equipped with a stir bar, thermometer, addition funnel (rubber septum) and N₂-line. Tetrahydrofuran (THF) (50 ml) was added, followed by dropwise addition of 4-(N-piperidino)-butyronitrile (10.0 g, 0.0658 mmol) in 50 ml of THF.

The reaction mixture was kept at room temperature overnight. The solution was quenched by the careful addition of 2.5 ml of water, 2.5 ml of sodium hydroxide, an additional 9 ml of water, filtered, washed with brine, dried over magnesium sulfate and concentrated under reduced pressure to give 7.7 g of an orange oil.

Next, 4-methylvaleronitrile (10.0 g, 0.103 mmol) was dissolved in ether, treated with methanol (3.5 g, 0.111 mmol) and cooled to 0°C. Anhydrous hydrochloric acid was introduced until saturation and the reaction mixture stirred at room temperature under N₂-atmosphere overnight.

The orange-pink reaction mixture was concentrated under reduced pressure, triturated with ether and the resulting off-white solid collected by vacuum filtration and air-dried to give 4.3 g of product.

Next, methyl 4-methylvalerylimidate (2.12 g, 0.0128 mmol) and 4-piperidino-1-butylamine (2.0 g, 0.0128 mmol) were mixed in 25 ml anhydrous methanol and stirred overnight under nitrogen atmosphere.

The reaction mixture was treated with 5N sodium hydroxide, extracted with ether (3 x 25 ml), dried over magnesium sulfate/potassium carbonate and then concentrated under reduced pressure to give 1.0 g of N-(4-piperidinobutyl)-4-methylvalerylamidine as a yellow oil.

### EXAMPLE 6

Octyl cyanide (10.0 g, 0.0718 mmol) and 50 ml ethyl ether were mixed in 2.56 g (0.08 mmol) methanol and cooled to 0°C, then saturated with hydrochloric acid and allowed to stir overnight.

The reaction mixture, which contained a small amount of precipitate, was concentrated under reduced pressure, triturated with ether, filtered and air dried to give 9.44 g of a white fluffy solid (63.6%), m.p. 90-92°C.

The above product, methyl octylimidate hydrochloride (8.44 g, 0.0406 mmol), was mixed with 3.5 g (0.0406 mmol) piperidine in 50 ml methanol and allowed to stand at room temperature overnight (CaCl₂ tube). The reaction mixture was concentrated under reduced pressure, triturated with ether and the resulting white solid collected by vacuum filtration and washed with ether, then air-dried to give 8.54 g of a white oily solid (85.2%).

Recrystallization with ethyl acetate gave 3.4 g of piperidine octylamidine hydrochloride as white crystals (m.p. 123-125°C). High resolution mass spectroscopy calculated for C₁₃H₂₀N₂: 210.2096. Found: 210.2087.

### EXAMPLE 7

### PREPARATION OF N-(1-TRIFLUOROMETHYL-UNDECANE)-PIPERIDINE

### Preparation of Magnesium Bromide Trifluoroacetate (1)

To a dry flask containing trifluoroacetic acid (15 g, 0.132 mmol) and anhydrous ether (50 ml) was added a solution of ethylmagnesium bromide (66 ml of a 2 M solution in tetrahydrofuran, 0.132 mmol) in anhydrous ether (50 ml) at -5°C under nitrogen atmosphere. The reaction mixture was allowed to warm to room temperature.

### Preparation of Decanylmagnesium Bromide (2)

To a dry flask containing magnesium (2.64 g, 0.11 mmol) and anhydrous ether (50 ml) was added a solution of 1-bromodecane (24.3 g, 0.11 mmol) in anhydrous ether (50 ml) under nitrogen atmosphere. The reaction mixture was stirred at room temperature until the magnesium had dissolved.

### Preparation of Trifluoromethyl-2-Dodecanone (3)

To the flask containing magnesium bromide trifluoroacetate (1) was added the solution of decanylmagnesium bromide (2) at -5°C under nitrogen atmosphere. The reaction mixture was stirred for 1 hour at room temperature, refluxed for 12 hours, cooled to 0°C and hydrolyzed with the dropwise addition of 5N hydrochloric acid (50 ml). The layers were separated, the aqueous extracted with ethyl acetate (2 x 20 ml), the combined organics washed with cold saturated sodium bicarbonate, then brine, and dried over magnesium sulfate. Evaporation gave 14.1 g of yellow oil which was purified by distillation providing trifluoromethyl-2-dodecanone (3) as a clear oil (10.1 g, 38%), b.p. 125°C @ 13.3 Pa (0.1 mm Hg). ¹H-NMR (300 MHz, CDCl₃) δ 0.85 (3H, t), 1.30 (14H, br s), 1.65 (2H, m), 2.70 (2H, t); ¹⁹F-NMR (CDCl₃) δ-80.02 (s); MS (CI/CH₄) 239 (M+H), 169 (M+H - HCF₃).

### Preparation of N-(1-Trifluoromethyl-Undecane)-Piperidine (4)

To a dry flask containing trifluoromethyl-2-dodecanone (3) (4.0g, 16.8 mmol), piperidine hydrochloride (1.84 g, 15.1 mmol), triethylamine (5 g, 50.4 mmol), and anhydrous methylene chloride (80 ml) at 10°C under nitrogen atmosphere was added titanium tetrachloride (8.4 ml of a 1 M solution in methylene chloride, 8.4 mmol) dropwise over 10 minutes. The reaction mixture was stirred at room temperature for 48 hours, then carefully quenched with a methanolic solution of sodium cyanoborohydride (3.4 g, 50.4 mmol in 20 ml methanol). The reaction mixture was stirred for 1 hour, carefully taken to pH 1 with 5N hydrochloric acid, stirred for 30 minutes, then taken to pH 13 with 5N sodium hydroxide. The desired product was extracted with ethyl acetate (3 x 75 ml), dried over magnesium sulfate, and evaporated providing 4.5 g orange oil. The oil was dissolved in ether, filtered, and treated with anhydrous hydrochloric acid. The white solid was collected and recrystallized (ethyl acetate/isopropyl alcohol) providing N-(1-trifluoromethyl-undecane)-piperidine (4) as a white crystalline solid (740 mg); m.p. 124-125°C, ¹H-NMR (300 MHz, DMSO-d₆, 90°C) 80.85 (3H,t), 1.30 (14H, br s), 1.4-1.9 (10H, m), 2.5 (1H, m), 2.75 (1H, m), 2.95 (1H,m); ¹⁹F-NMR (DMSO-d₆, 120°C) 8-67 (br s); MS (CI/CH₄) 308 (M+H), 306 (M-H), 288 (M+H-HF); High Resolution MS (FT) Anal. Calcd. for C₁₇H₃₂F₃N: 307.249. Found: 307.246.

## Claims (Claims for the following Contracting State(s): AT, BE, CH, DE, DK, FR, GB, IT, LI, LU, NL, SE)

1. A compound according to the formula (A): 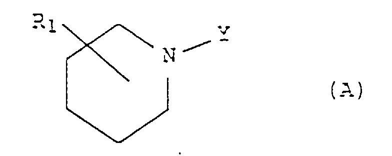 and the pharmaceutically acceptable salts thereof wherein Y is -A-(Alk₁)ₓ-D-(Alk₂)-E-(Alk₃)_{y}-CH₃ wherein, the moiety A is -CH₂- or 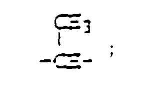 D and E are each independently 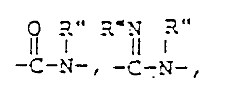 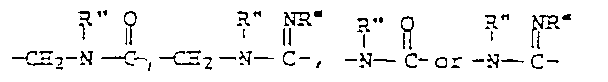 or a direct bond, with the proviso that when D is a moiety from the group 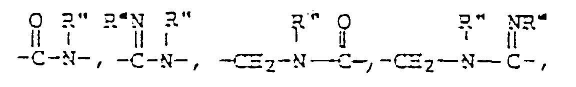 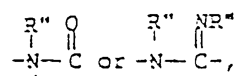 E cannot be a moiety from the same group, and that D and E cannot both be direct bonds;
(Alk₁), (Alk₂) and (Alk₃) are each independently a straight chain alkylene moiety containing up to 5 carbon atoms, optionally substituted with up to 3 methyl groups, or
(Alk₁), (Alk₂) and (Alk₃) are each independently a straight chain alkenylene moiety containing from 2 to 6 carbon atoms, the straight chain alkenylene moiety having 1 to 2 double bonds, and optionally substituted with up to 3 methyl groups;
R₁ is hydrogen, hydroxy or C₁₋₄ lower alkyl;
R', R" and R"' are each independently hydrogen or C₁₋₄ lower alkyl; and x and y independently represent 0 or 1; with the proviso that when A is -CH₂- or 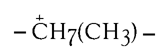 and one of D or E is a direct bond, the other of D and E is not -C(O)N(R")- except when the compound of formula (A) is N-(3-methylbutyl)-1-piperidine propanamide, N-butyl-N-methyl-1-piperidine butyramide or 4-(3-methylbutylamino)-1-piperidinobutane and the pharmaceutically acceptable salts thereof.

2. A compound according to Claim 1 which is N-(3-piperidinopropyl)-4-methylvalerylamidine, and the pharmaceutically acceptable salts thereof.

3. A compound according to Claim 1 which is N-(4-piperidinobutyl)-4-methylvalerylamidine, and the pharmaceutically acceptable salts thereof.

4. A compound according to Claim 1 which is piperidine octylamidine, and the pharmaceutically acceptable salts thereof.

5. A compound according to Claim 1 which is N-(1-trifluoromethyl-undecane)-piperidine.

6. A process for preparing a compound according to any one of Claims 1 to 5 comprising carrying out any of the following process steps (A) to (F) :
(A) for those compounds according to Formula A wherein D is -C(O)N(R")-, A is -CH₂- or -CH(CH₃)-, E is a direct bond, y is 0, and (Alk₁), (Alk₂), R" and x are as defined above in Formula A, carrying out the reaction depicted in Reaction Scheme I by (1) conducting the N-alkylation of piperidine 2 by a bomoalkyl ester 1, to produce Intermediate I and (2) conducting an amidation reaction between Intermediate I and a substituted amine 3 to produce a compound of Formula I: 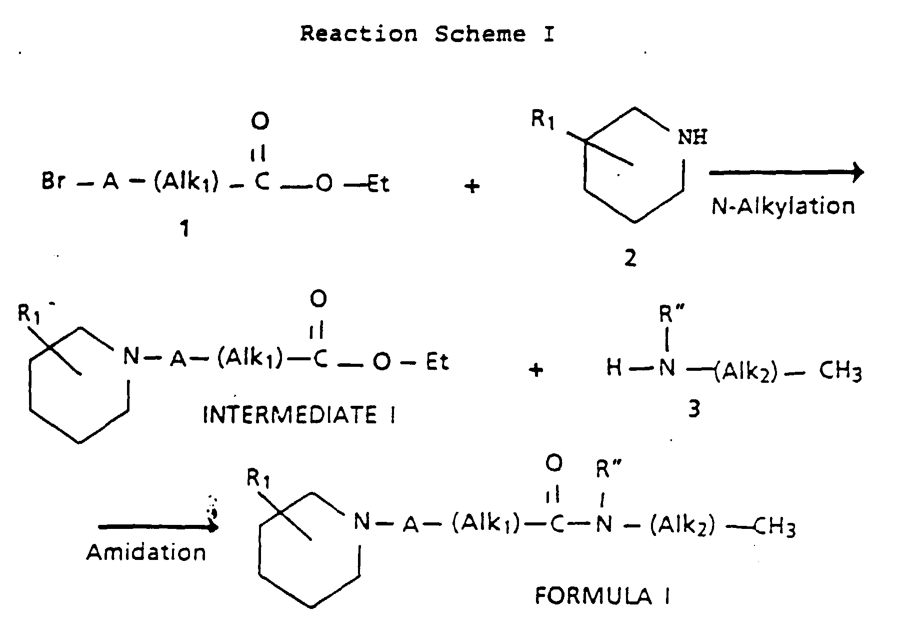
(B) for those compounds according to Formula A wherein D is -C(=NR"')-N(R")-, E is a direct bond and (Alk₃) is an alkylene moiety of 0 carbon atoms and the other substituents are as first defined in Formula A, carrying out the reaction depicted in Reaction Scheme III by (1) conducting an alkylation of the amide of Formula I with an alkylating agent, such as triethyloxonium tetrafluoroborate and (2) conducting a displacement reaction between the alkylated amide and an excess amount of the desired amine to produce a compound of Formula III: 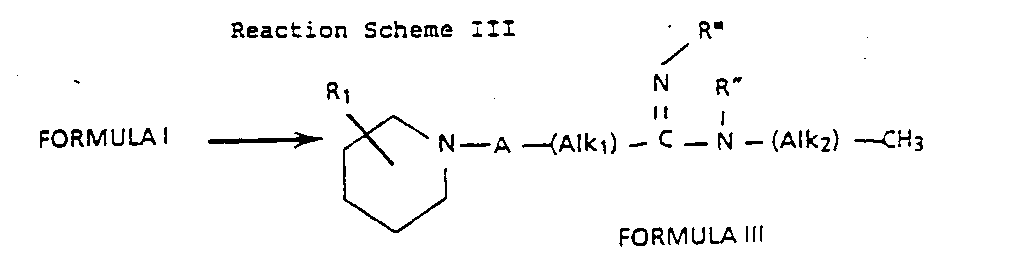
(C) for those compounds according to Formula A wherein D is -CH₂-N (R")-C(=NR"')-, R"' is hydrogen, the moiety A is -CH₂- or 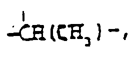 E is a direct bond, y is 0, and (Alk₁), (Alk₂), R₁, R" and x are as first defined in Formula A, carrying out the reaction depicted in Reaction Scheme IV by, (1) conducting a Pinner reaction in which alkylnitrile 1, in wherein Alk₂, has the same definition as that desired in the final product, is converted to imidate ester 3 and (2) conducting an amidation reaction between imidate ester 3 and Intermediate II to produce a compound of Formula IV : 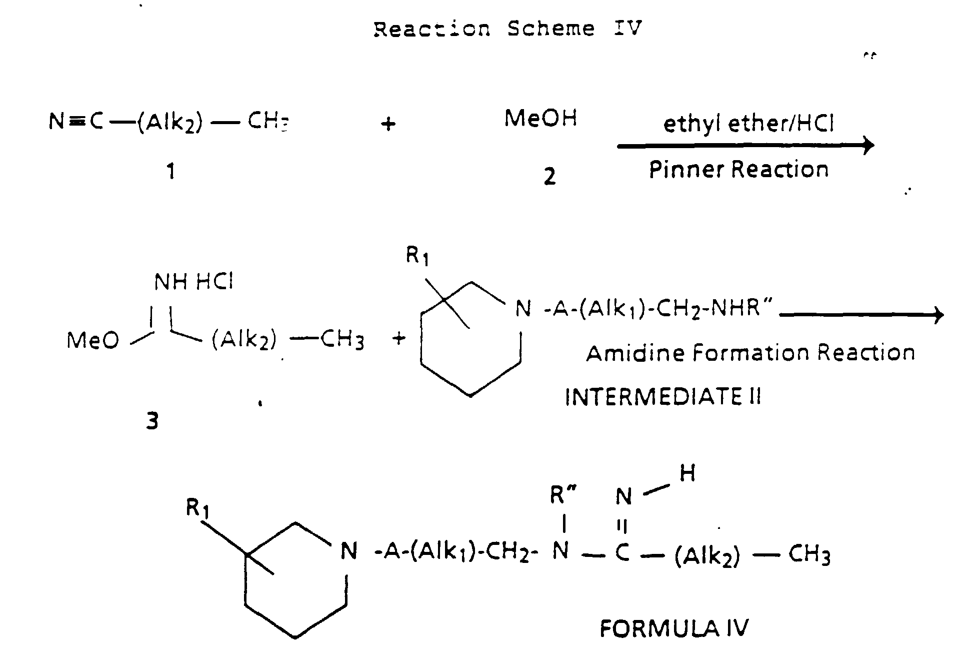 whereby Intermediate II, wherein R" is hydrogen (Intermediate IIA), is obtainable by conducting a reduction of the alkylcyano piperidine IA, wherein A and Alk₁ have the same definition as that desired in the final product, with a reducing agent, such as lithium aluminum hydride: 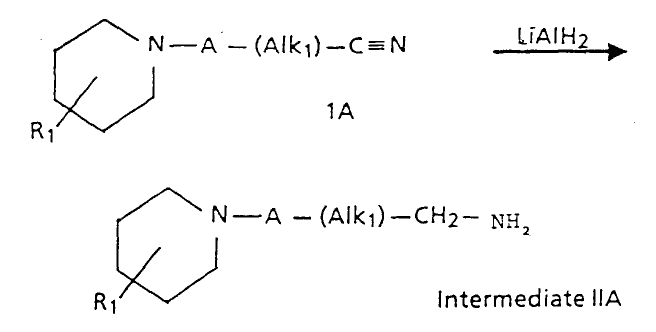 and whereby Intermediate II, wherein R" is C₁-C₄ lower alkyl (Intermediate IIB), is obtainable as follows: 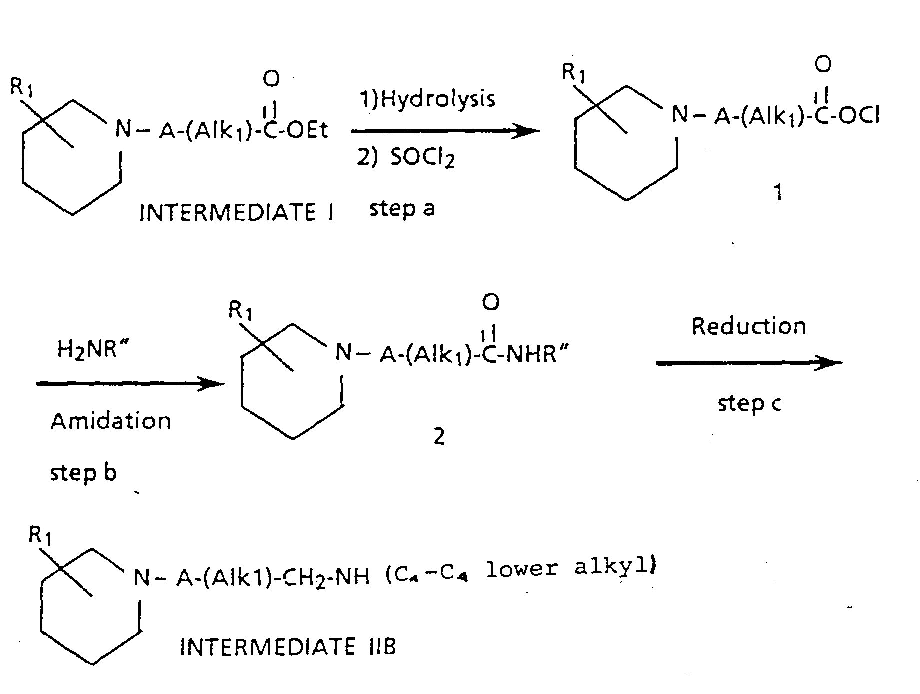
(D) for those compounds according to Formula A wherein D is -CH₂-N(R")-C(O)-, E is a direct bond, y is 0, and the other substituents are as first defined in Formula A, carrying out the reaction depicted in Reaction Scheme V by conducting an acidic hydrolysis of the amidine of Formula IV to produce the amide of Formula V: 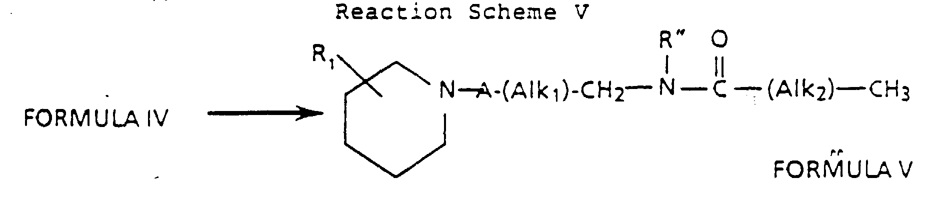
(E) for those compounds according to Formula A wherein D is -CH₂-N(R")-C(=NR"')-, E is a direct bond, y is 0 and the other substituents are as first defined in Formula A, carrying out the reaction depicted in Reaction Scheme VI by (1) conducting an alkylation of the amide of Formula V with an alkylating agent, such as triethyloxonium tetrafluoroborate and (2) conducting a displacement reaction between the alkylated amide and an excess of a desired amine to produce a compound of Formula VI: 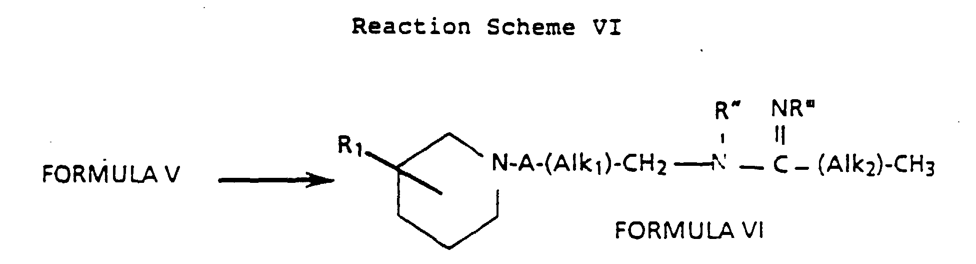
(F) for those compounds according to Formula A wherein E is -C(=NR"')-N(R")-, and the other substituents are as first defined in Formula (A) by carrying out the reaction depicted in Reaction Scheme X by (1) conducting an alkylation of the amide of Formula IX with an alkylating agent, such as triethyloxonium tetrafluoroborate and (2) conducting a displacement reaction between the alkylated amide and an excess of substituted amine H₂NR'", wherein R'" has the same definition as desired in the final product, to produce a compound of Formula X:

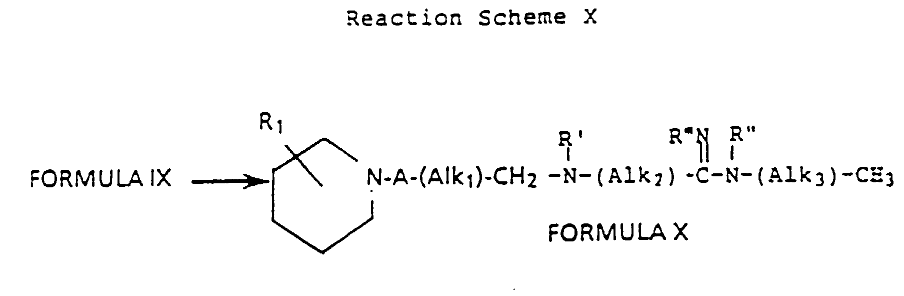

7. Use of a compound according to any of Claims 1 to 5 for the preparation of a pharmaceutical composition for lowering plasma cholesterol in mammals.

8. Use of a compound according to any of Claims 1 to 5 for the preparation of a pharmaceutical composition for the treatment of fungal infections in animals.

9. A pharmaceutical composition comprising a compound according to any of Claims 1 to 5 present in an effective amount in admixture with an inert carrier.

10. A pharmaceutical composition comprising a compound according to any of Claims 1 to 5 present in an effective amount in admixture with a pharmaceutically acceptable carrier.

11. A pharmaceutical composition according to Claim 9 or 10 for lowering plasma cholesterol in mammals.

12. A pharmaceutical composition according to Claim 9 or 10 for treating fungal infections in animals.

## Claims (Claims for the following Contracting State(s): ES)

1. A process for preparing a compound having Formula A: 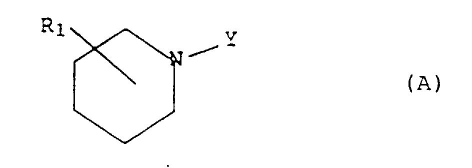 and the pharmaceutically acceptable salts thereof wherein Y is -A-(Alk₁)ₓ-D-(Alk₂)-E-(Alk₃)_{y}-CH₃ wherein, the moiety A is -CH₂- or 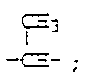 D and E are each independently 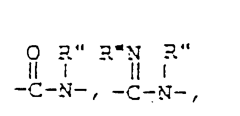 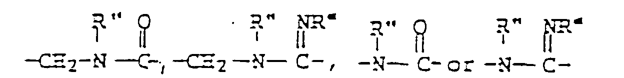 or a direct bond, with the proviso that when D is a moiety from the group 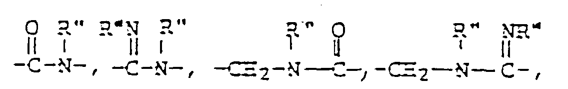 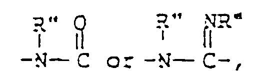 E cannot be a moiety from the same group, and that D and E cannot both be direct bonds;
(Alk₁), (Alk₂) and (Alk₃) are each independently a straight chain alkylene moiety containing up to 5 carbon atoms, optionally substituted with up to 3 methyl groups, or
(Alk₁), (Alk₂) and (Alk₃) are each independently a straight chain alkenylene moiety containing from 2 to 6 carbon atoms, the straight chain alkenylene moiety having 1 to 2 double bonds, and optionally substituted with up to 3 methyl groups;
R₁ is hydrogen, hydroxy or C₁₋₄ lower alkyl;
R', R" and R"' are each independently hydrogen or C₁₋₄ lower alkyl; and x and y independently represent 0 or 1; with the proviso that when A is -CH₂- or 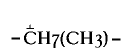 and one of D or E is a direct bond, the other of D and E is not -CH₂-N(R')-, -N(R')-, or -C(O)N(R")- except when the compound of formula (A) is N-(3-methylbutyl)-1-piperidine propanamide, N-butyl-N-methyl-1-piperidine butyramide or 4-(3-methylbutylamino)-1-piperidinobutane and the pharmaceutically acceptable salts thereof
comprising carrying out any of the following process steps (A) to (F) :
(A) for those compounds according to Formula A wherein D is -C(O)N(R")-, A is -CH₂- or -CH(CH₃)-, E is a direct bond, y is 0, and (Alk₁), (Alk₂), R", and x are as defined above in Formula A, carrying out the reaction depicted in Reaction Scheme I by (1) conducting the N-alkylation of piperidine 2 by a bomoalkyl ester 1, to produce Intermediate I and (2) conducting an amidation reaction between Intermediate I and a substituted amine 3 to produce a compound of Formula I: 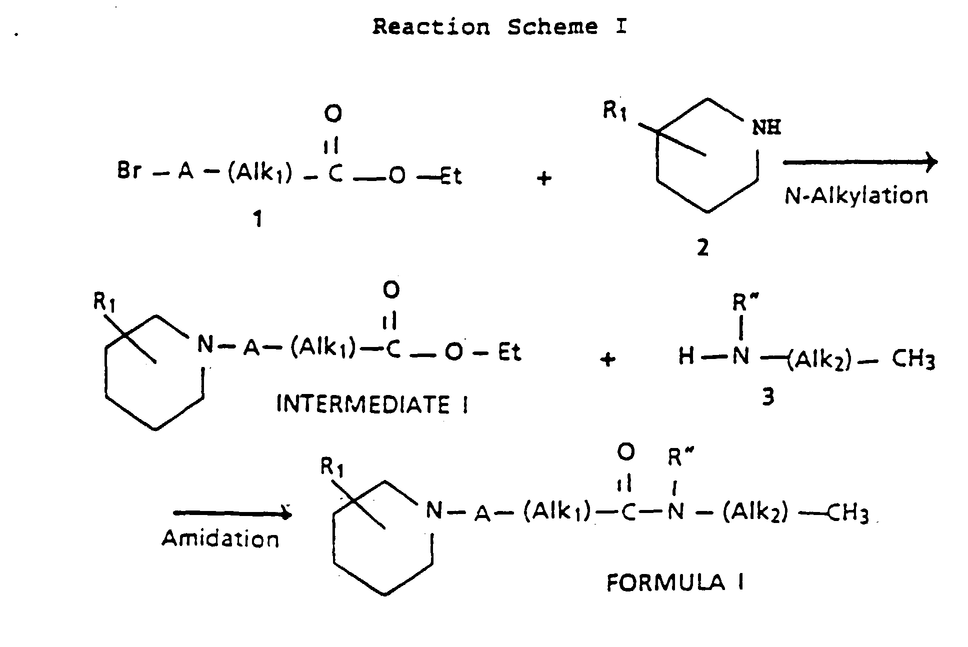 -CH₂(NR')-, the moiety A is -CH₂- or 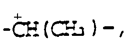 E is a direct bond, y is 0, and (Alk₁), (Alk₂), R₁, R' and x are as first defined in Formula A, carrying out the reaction depicted in Reaction Scheme II by conducting a reduction of the amide of Formula I with a reducing agent,
(B) for those compounds according to Formula A wherein D is -C(=NR"')-N(R")- E is a direct bond and (Alk₃) is an alkylene moiety of 0 carbon atoms and the other substituents are as first defined in Formula A, carrying out the reaction depicted in Reaction Scheme III by (1) conducting an alkylation of the amide of Formula I with an alkylating agent, such as triethyloxonium tetrafluoroborate and (2) conducting a displacement reaction between the alkylated amide and an excess amount of the desired amine to produce a compound of Formula III: 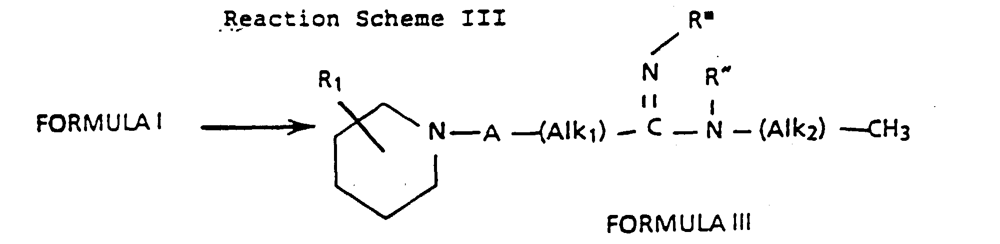
(C) for those compounds according to Formula A wherein D is -CH₂-N(R")-C(=NR"')-, R"' is hydrogen, the moiety A is -CH₂- or 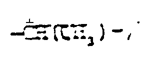 E is a direct bond, y is 0, and (Alk₁), (Alk₂), R₁, R" and x are as first defined in Formula A, carrying out the reaction depicted in Reaction Scheme IV by, (1) conducting a Pinner reaction in which alkylnitrile 1, in wherein Alk₂, has the same definition as that desired in the final product, is converted to imidate ester 3 and (2) conducting an amidation reaction between imidate ester 3 and Intermediate II to produce a compound of Formula IV: 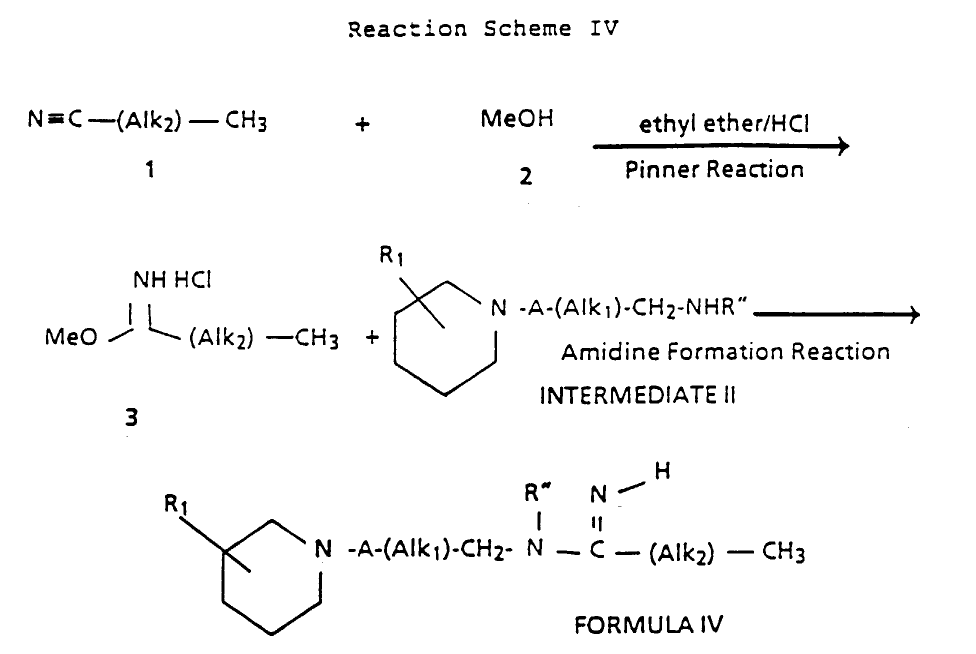 whereby Intermediate II, wherein R" is hydrogen (Intermediate IIA), is obtainable by conducting a reduction of the alkylcyano piperidine IA, conducting a reduction of the alkylcyano piperidine IA, wherein A and Alk₁ have the same definition as that desired in the final product, with a reducing agent, such as lithium aluminum hydride: 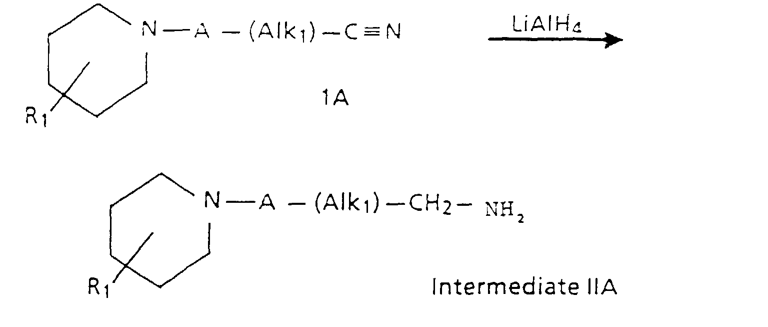 and whereby Intermediate II, wherein R" is C₁-C₄ lower alkyl (Intermediate IIB), is obtainable as follows: 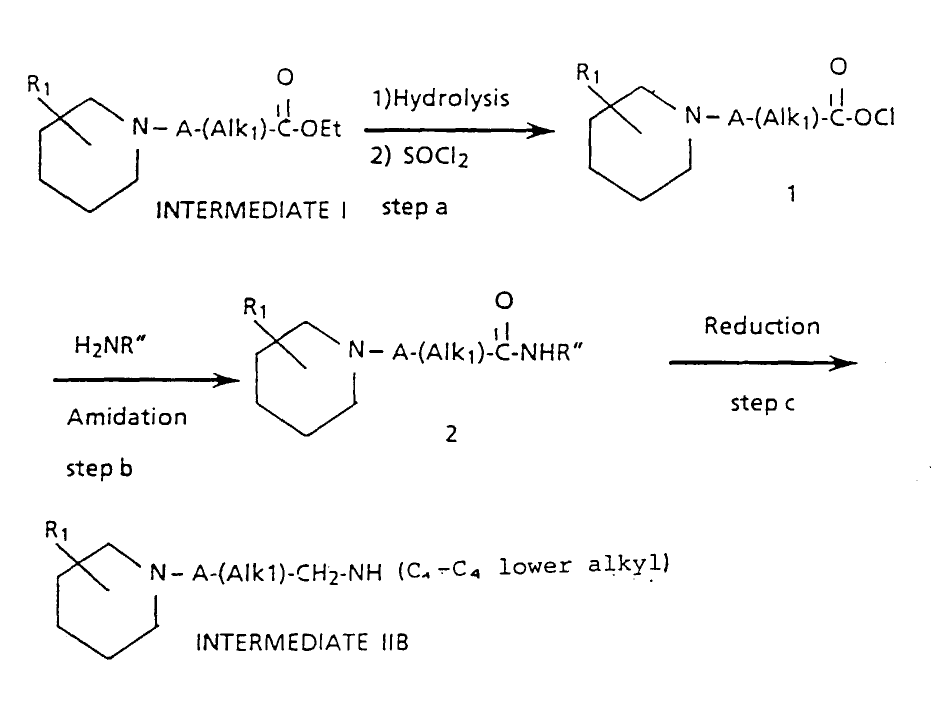
(D) for those compounds according to Formula A wherein D is -CH₂-N(R")-C(O)-, E is a direct bond, y is 0, and the other substituents are as first defined in Formula A, carrying out the reaction depicted in Reaction Scheme V by conducting an acidic hydrolysis of the amidine of Formula IV to produce the amide of Formula V: 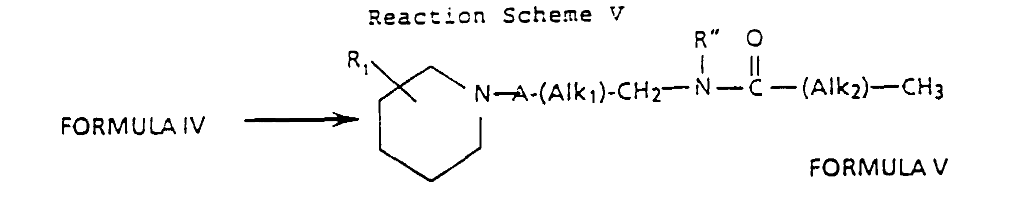
(E) for those compounds according to Formula A wherein D is -CH₂-N(R")-C(=NR"')-, E is a direct bond is 0, and the other substituents are as first defined in Formula A, carrying out the reaction depicted in Reaction Scheme VI by (1) conducting an alkylation of the amide of Formula V with an alkylating agent, such as triethyloxonium tetrafluoroborate and (2) conducting a displacement reaction between the alkylated amide and an excess of a desired amine to produce a compound of Formula VI: 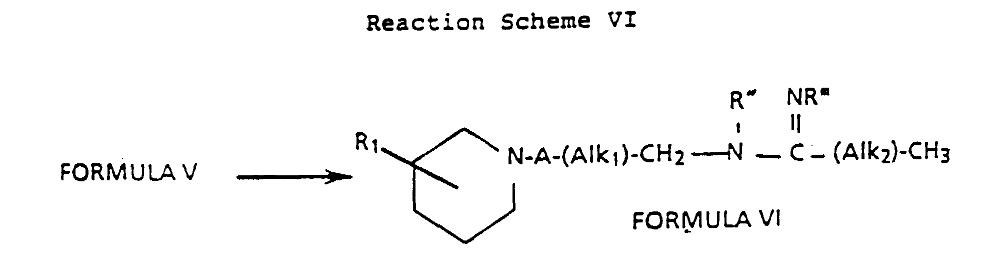
(F) for those compounds according to Formula A wherein E is -C(=NR'")-N(R")-, and the other substituents are as first defined in Formula A by carrying out the reaction depicted in Reaction Scheme X by (1) conducting an alkylation of the amide of Formula IX with an alkylating agent, such as triethyloxonium tetrafluoroborate and (2) conducting a displacement reaction between the alkylated amide and an excess of substituted amine H₂NR"', wherein R"' has the same definition as desired in the final product to produce a compound of Formula X:

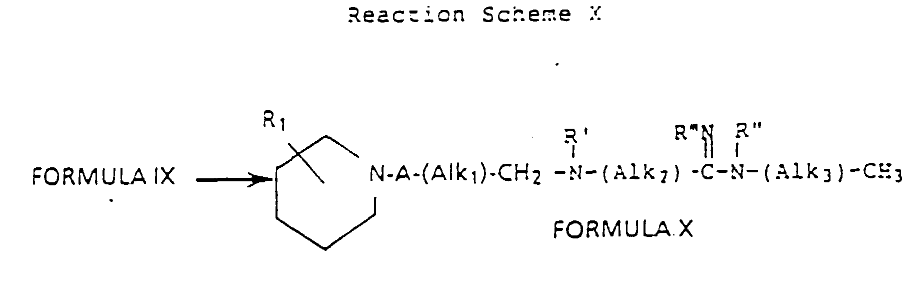

2. A process for preparing a compound according to Claim 1 which is N-(3-piperidinopropyl)-4-methylvalerylamidine, and the pharmaceutically acceptable salts thereof.

3. A process for preparing a compound according to Claim 1 which is N-(4-piperidinobutyl)-4-methylvalerylamidine, and the pharmaceutically acceptable salts thereof.

4. A process for preparing a compound according to Claim 1 which is piperidine octylamidine, and the pharmaceutically acceptable salts thereof.

5. A process for preparing a compound according to Claim 1 which is N-(1-trifluoromethyl-undecane)-piperidine.

## Claims (Claims for the following Contracting State(s): GR)

1. A compound according to the formula A: 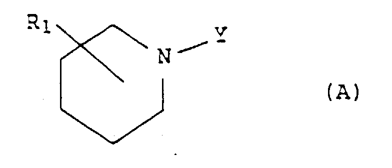 and the pharmaceutically acceptable salts thereof wherein Y is -A-(Alk₁)ₓ-D-(Alk₂)-E-(Alk₃)_{y}-CH₃ wherein, the moiety is -CH₂- or 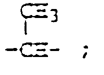 D and E are each independently 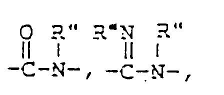 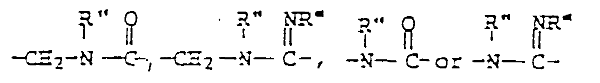 or a direct bond, with the proviso that when D is a moiety from the group 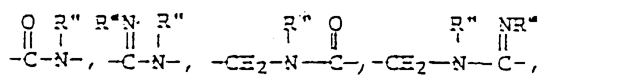 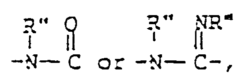 E cannot be a moiety on the same group, and that D and E cannot both be direct bonds;
(Alk₁), (Alk₂) and (Alk₃) are each independently a straight chain alkylene moiety containing up to 5 carbon atoms, optionally substituted with up to 3 methyl groups, or
(Alk₁), (Alk₂) and (Alk₃) are each independently a straight chain alkenylene moiety containing from 2 to 6 carbon atoms, the straight chain alkenylene moiety having 1 to 2 double bonds, and optionally substituted with up to 3 methyl groups;
R₁ is hydrogen, hydroxy or C₁₋₄ lower alkyl;
R', R" and R"' are each independently hydrogen or C₁₋₄ lower alkylₓ and x and y independently represent 0 or 1; with the proviso that when A is -CH₂- or 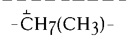 and one of D or E is a direct bond, the other D and E is not -CH₂N(R')-, -N(R')-, or -C(O)N(R")- except when the compound of formula (A) is N-(3-methylbutyl)-1-piperidine propanamide, N-butyl-N-methyl-1-piperidinebutyramide or 4-(3-methylbutylamino)-1-piperidinobutane and the pharmaceutically acceptable salts thereof.

2. A compound according to Claim 1 which is N-(3-piperidinopropyl)-4-methylvalerylamidine, and the pharmaceutically acceptable salts thereof.

3. A compound according to Claim 1 which is N-(4-piperidinobutyl)-4-methylvalerylamidine, and the pharmaceutically acceptable salts thereof.

4. A compound according to Claim 1 which is piperidine octylamidine, and the pharmaceutically acceptable salts thereof.

5. A compound according to Claim 1 which is N-(1-trifluoromethyl-undecane)-piperidine.

6. A process for preparing a compound according to any one of Claims 1 to 5 comprising carrying out any of the following process steps (A) to (F):
(A) for those compounds according to Formula A wherein D is -C(O)N(R")-, A is -CH₂- or 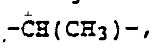 E is a direct bond, y is 0, and (Alk₁), (Alk₂), R" and x are as defined above in Formula A, carrying out the reaction depicted in Reaction Scheme I by (1) conducting the N-alkylation of piperidine 2 by a bomoalkyl ester 1, to produce Intermediate I and (2) conducting an amidation reaction between Intermediate I and a substituted amine 3 to produce a compound of Formula I: 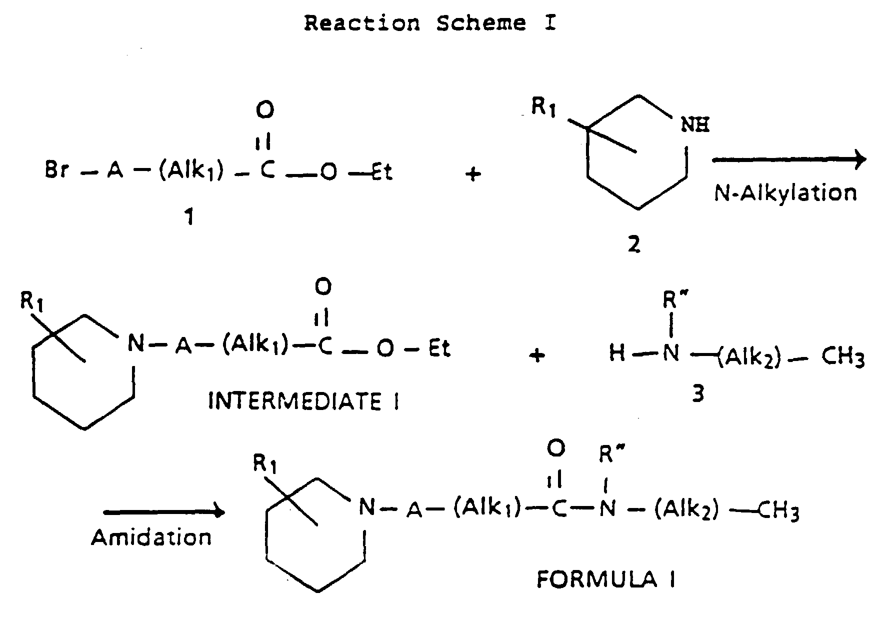
(B) for those compounds according to Formula A wherein D is -C(=NR"'-)-N(R")- E is a direct bond and (Alk₃) is an alkylene moiety of 0 carbon atoms and the other substituents are as first defined in Formula A , carrying out the reaction depicted in Reaction Scheme III by (1) conducting an alkylation of the amide of Formula I with an alkylating agent, such as triethyloxonium tetrafluoroborate and (2) conducting a displacement reaction between the alkylated amide and an excess amount of the desired amine to produce a compound of Formula III: 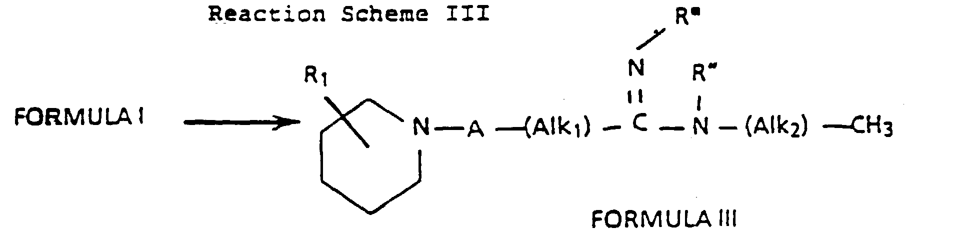
(C) for those compounds according to Formula A wherein D is -CH₂-N(R")-C(=NR"')-, R"' is hydrogen, the moiety A is -CH₂- or 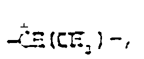 E is a direct bond, y is 0 and (Alk₁), (Alk₂), R₁, R" and x are as first defined in Formula A, carrying out the reaction depicted in Reaction Scheme IV by, (1) conducting a Pinner reaction in which alkylnitrile 1, in wherein Alk₂, has the same definition as that desired in the final product, is converted to imidate ester 3 and (2) conducting an amidation reaction between imidate ester 3 and Intermediate II to produce a compound of Formula IV: 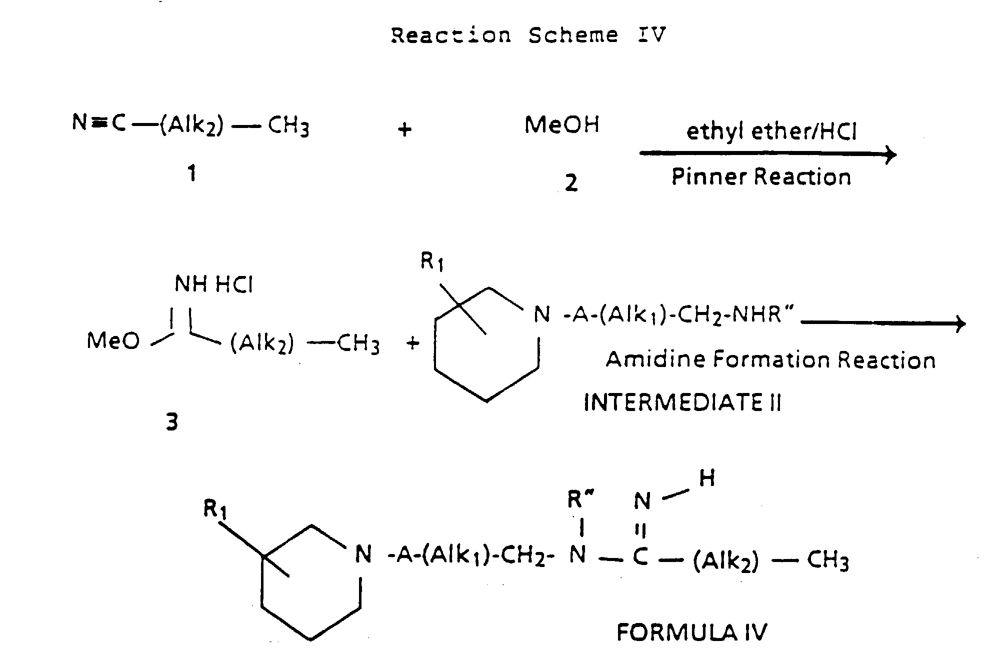 whereby Intermediate II, wherein R" is hydrogen (Intermediate IIA), is obtainable by conducting a reduction of the alkylcyano piperidine IA, conducting a reduction of the alkylcyano piperidine IA, wherein A and Alk₁ have the same definition as that desired in the final product, with a reducing agent, such as lithium aluminum hydride: 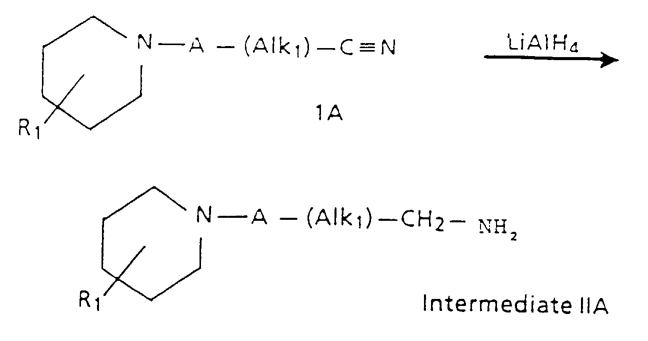 and whereby Intermediate II, wherein R" is C₁-C₄ lower alkyl (Intermediate IIB), is obtainable as follows: 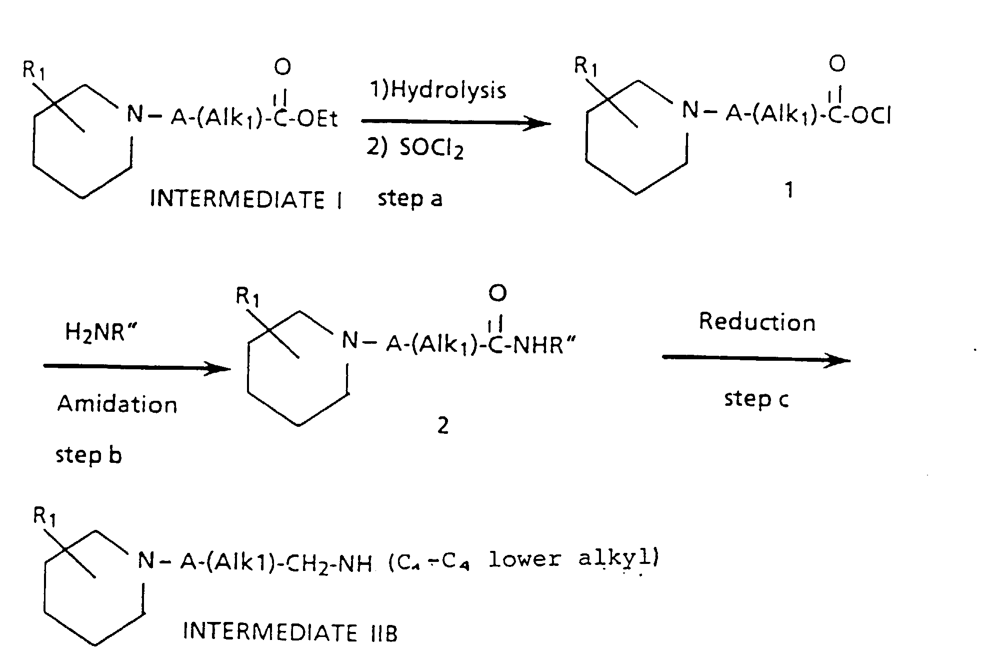
(D) for those compounds according to Formula A wherein D is -CH₂-N(R")-C(O)-, E is a direct bond, y is 0, and the other substituents are as first defined in Formula A, carrying out the reaction depicted in Reaction Scheme V by conducting an acidic hydrolysis of the amidine of Formula IV to produce the amide, of Formula V: 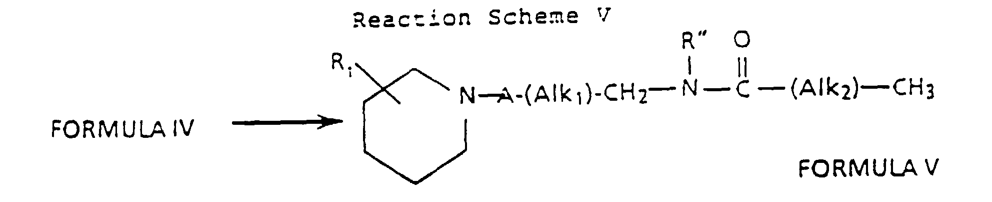
(E) for those compounds according to Formula A wherein D is -CH₂-N(R")-C(=NR"')-, E is a direct bond, y is 0, and the other substituents are as first defined in Formula A, carrying out the reaction depicted in Reaction Scheme VI by (1) conducting an alkylation of the amide of Formula V with an alkylating agent, such as triethyloxonium tetrafluoroborate and (2) conducting a displacement reaction between the alkylated amide and an excess of a desired amine to produce a compound of Formula VI: 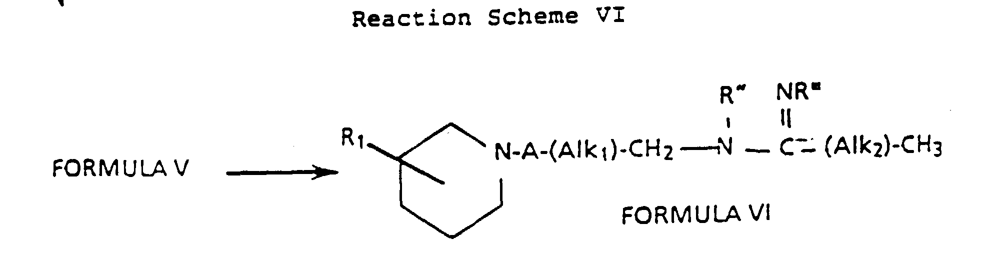
(F) for those compounds according to Formula A wherein E is -C(=NR'")-N(R")-, and the other substituents are as first defined in Formula (A) by carrying out the reaction depicted in Reaction Scheme X by (1) conducting an alkylation of the amide of Formula IX with an alkylating agent, such as triethyloxonium tetrafluoroborate and (2) conducting a displacement reaction between the alkylated amide and an excess of substituted amine H₂NR"', wherein R"' has the same definition as desired in the final product, to produce a compound of Formula X :

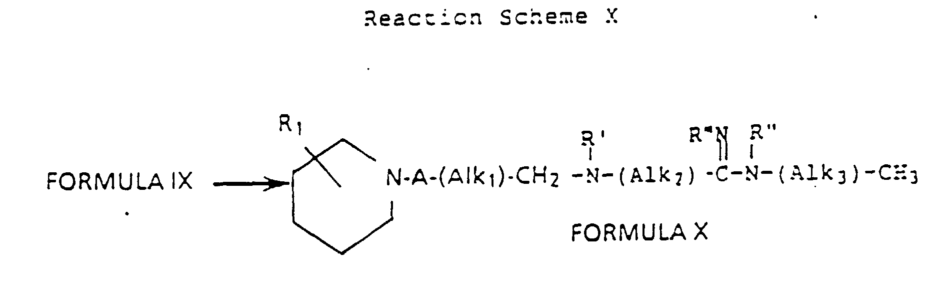

7. Use of a compound according to any of Claims 1 to 5 for the preparation of a pharmaceutical composition for lowering plasma cholesterol in mammals.

8. Use of a compound according to any of Claims 1 to 5 for the preparation of a pharmaceutical composition for the treatment of fungal infections in animals.

## Patentansprüche (Patentansprüche für folgende(n) Vertragsstaat(en): AT, BE, CH, DE, DK, FR, GB, IT, LI, LU, NL, SE)

1. Verbindung, wiedergegeben durch Formel (A): 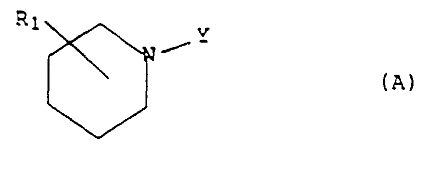 und die pharmazeutisch verträglichen Salze davon, wobei Y -A-(Alk₁)ₓ-D-(Alk₂)-E-(Alk₃)_{y}-CH₃ bedeutet, wobei der Rest A -CH₂- oder 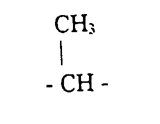 bedeutet;
D und E jeweils unabhängig 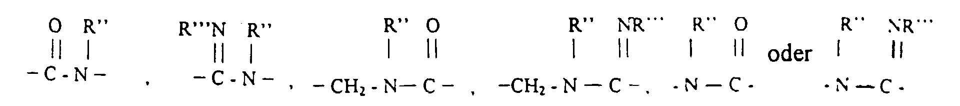 oder eine direkte Bindung bedeuten, mit der Maßgabe, daß wenn D einen Rest aus der Gruppe 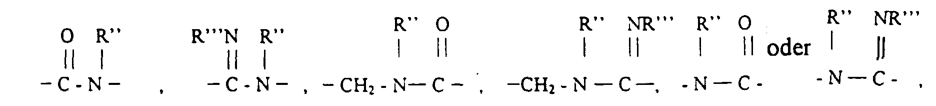 bedeutet, E nicht ein Rest aus der gleichen Gruppe sein kann; und daß D und E nicht beide direkte Bindungen sein können; (Alk₁), (Alk₂) und (Alk₃) bedeuten jeweils unabhängig einen geradkettigen Alkylenrest mit bis zu 5 Kohlenstoffatomen, der gegebenenfalls mit bis zu 3 Methylgruppen substituiert ist, oder (Alk₁), (Alk₂) und (Alk₃) bedeuten jeweils unabhängig einen geradkettigen Alkenylenrest mit 2 bis 6 Kohlenstoffatomen, wobei der geradkettige Alkenylenrest 1 bis 2 Doppelbindungen besitzt und gegebenenfalls mit bis zu 3 Methylgruppen substituiert ist; R₁ bedeutet ein Wasserstoffatom, eine Hydroxygruppe oder einen niederen C₁₋₄ Alkylrest;
R', R" und R"' bedeuten jeweils unabhängig ein Wasserstoffatom oder einen niederen C₁₋₄ Alkylrest; und x und y bedeuten unabhängig 0 oder 1; mit der Maßgabe, daß wenn A -CH₂- oder -CH(CH₃)- bedeutet und einer der Reste D oder E eine direkte Bindung ist, der andere der Reste D und E nicht -C(O)N(R")- sein kann, ausgenommen wenn die Verbindung der Formel (A) N-(3-Methylbutyl)1-piperidinpropanamid, N-Butyl-N-methyl-1-piperidinbutyramid oder 4-(3-Methylbutylamino)-1-piperidinobutan und die pharmazeutisch verträglichen Salze davon darstellt.

2. N-(3-Piperidinopropyl)-4-methylvalerylamidin nach Anspruch 1 und die pharmzeutisch verträglichen Salze davon.

3. N-(4-Piperidinobutyl)-4-methylvalerylamidin nach Anspruch 1 und die pharmazeutisch verträglichen Salze davon.

4. Piperidinoctylamidin nach Anspruch 1 und die pharmazeutisch verträglichen Salze davon.

5. N-(1-Trifluormethylundecan)-piperidin nach Anspruch 1.

6. Verfahren zur Herstellung einer Verbindung nach einem der Ansprüche 1 bis 5, umfassend die Durchführung eines der nachstehenden Verfahrensschemata (A) bis (F):
(A) für die Verbindungen, wiedergegeben durch Formel A, in der D -C(O)N(R")- ist, A -CH₂- oder -CH(CH₃)- bedeutet, E eine direkte Bindung darstellt, y 0 ist und (Alk₁), (Alk₂), R" und x die vorstehenden Bedeutungen der Formel A besitzen, Durchführung der Umsetzung nach Reaktionsschema I durch (1) N-Alkylierung von Piperidin 2 durch einen Bromalkylester 1, wobei das Zwischenprodukt I hergestellt wird und (2) Durchführung einer Amidierungsreaktion zwischen dem Zwischenprodukt I und einem substituierten Amin 3, wobei eine Verbindung der Formel I hergestellt wird: 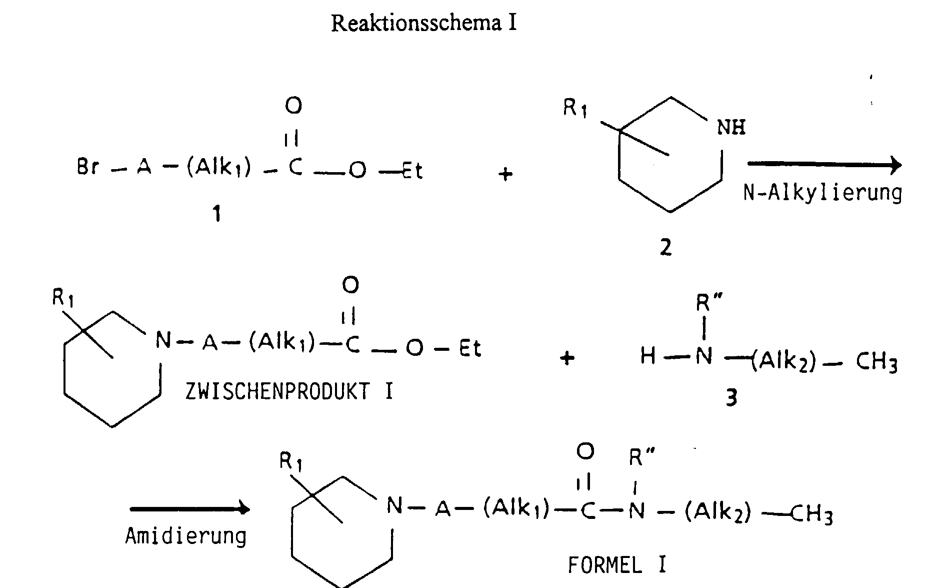
(B) für die Verbindungen, wiedergegeben durch Formel A, in der D -C(=NR"')-N(R")- ist, E eine direkte Bindung darstellt und (Alk₃) einen Alkylenrest mit 0 Kohlenstoffatomen bedeutet und die anderen Substituenten die gleichen Bedeutungen besitzen, wie zuerst in Formel A beschrieben, Durchführung der im Reaktionsschema III dargestellten Umsetzung durch (1) Alkylierung des Amids der Formel I mit einem Alkylierungsmittel, wie Triethyloxoniumtetrafluoroborat und (2) Durchführung einer Austauschreaktion zwischen dem alkylierten Amid und einem Überschuß des gewünschten Amins, wobei eine Verbindung der Formel III hergestellt wird: 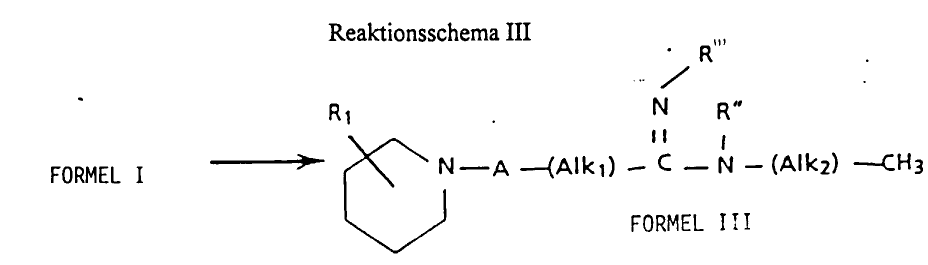
(C) für die Verbindungen, wiedergegeben durch Formel A, wobei D -CH₂-N(R")-C(=NR"')- bedeutet, R"' ein Wasserstoffatom bedeutet, der Rest A -CH₂- oder -CH(CH₃)- bedeutet, E eine direkte Bindung darstellt, y 0 ist und (Alk₁), (Alk₂), R₁, R" und x die gleiche Bedeutung besitzen, wie zuerst in Formel A beschrieben wurde, Durchführung der im Reaktionsschema IV beschriebenen Umsetzung durch (1) eine Pinner-Reaktion, wobei das Alkylnitril 1, wobei (Alk₂) die gleiche Bedeutung besitzt, wie sie im Endprodukt gewünscht wird, zum Imidatester 3 umgewandelt wird und (2) Durchführung einer Amidierungsreaktion zwischen dem Imidatester 3 und dem Zwischenprodukt II, wobei eine Verbindung der Formel IV hergestellt wird: 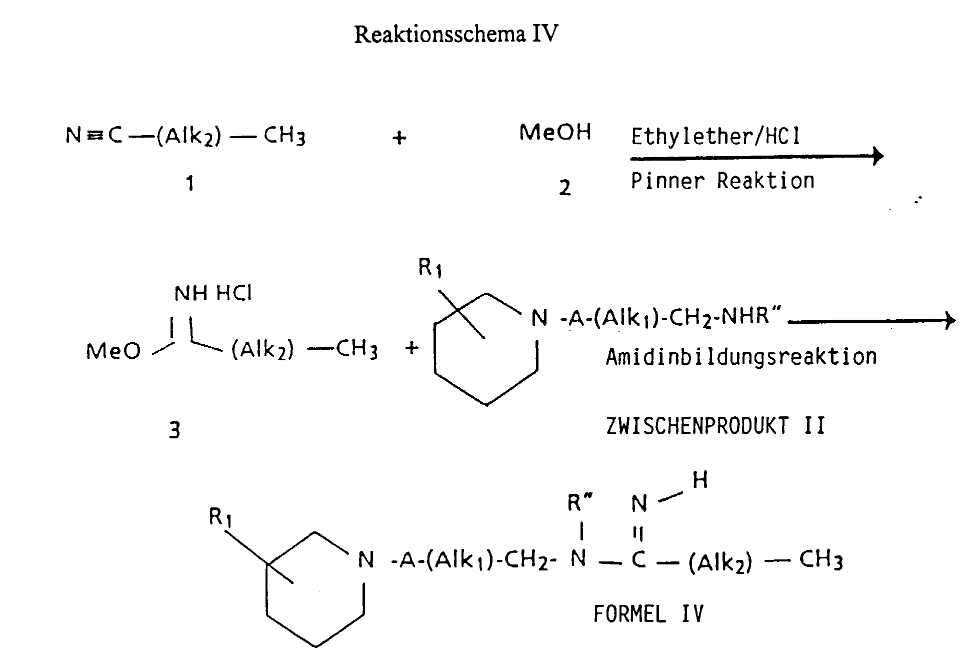 wodurch das Zwischenprodukt II erhältlich ist, in dem R" ein Wasserstoffatom bedeutet (Zwischenprodukt IIA), durch Reduktion des Alkylcyanopiperidins IA, in dem A und Alk₁ die gleiche Bedeutung besitzen, wie sie für das Endprodukt gewünscht werden, mit einem Reduktionsmittel, wie Lithiumaluminiumhydrid: 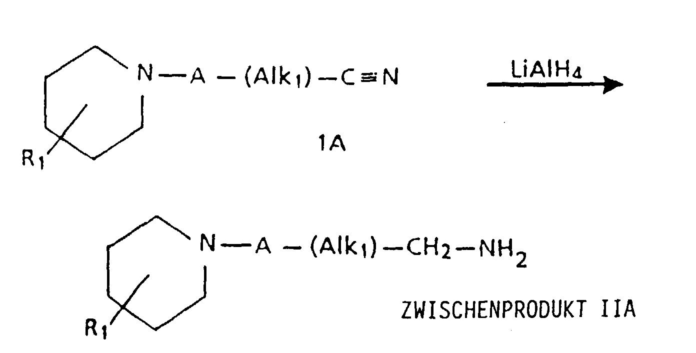 und wobei das Zwischenprodukt II, wobei R" einen niederen C₁-C₄ Alkylrest (Zwischenprodukt IIB) bedeutet, wie nachstehend wiedergegeben erhältlich ist: 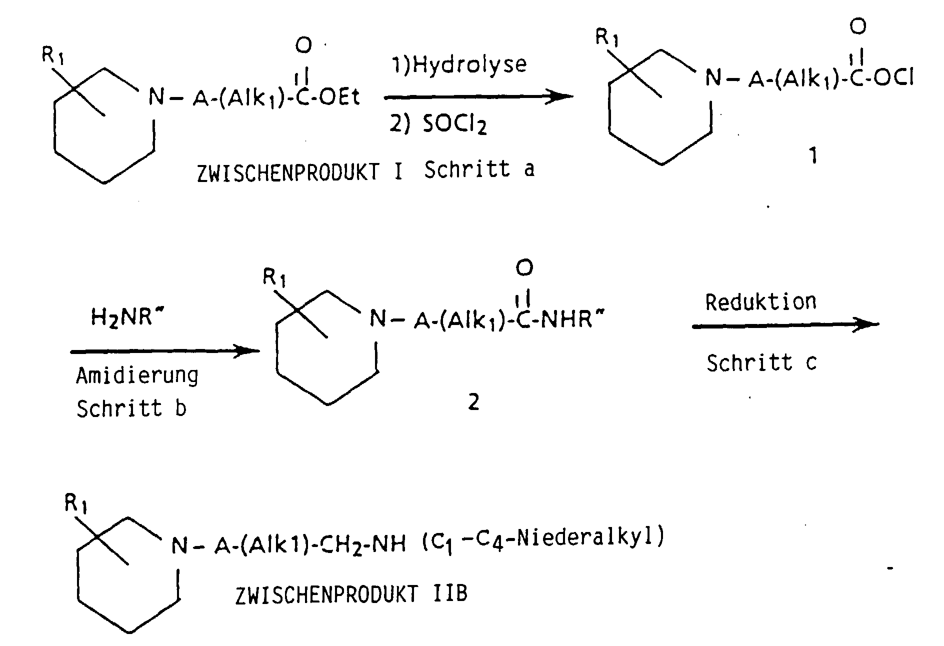
(D) für die Verbindungen, wiedergegeben durch Formel A, in der D -CH₂-N(R")-C(O)- ist, E eine direkte Bindung darstellt, y 0 ist und die anderen Substituenten die gleiche Bedeutung besitzen, wie sie zuerst in Formel A beschrieben sind, Durchführung der im Reaktionsschema V dargestellten Umsetzung durch saure Hydrolyse des Amidins der Formel IV, wobei das Amid der Formel V hergestellt wird: 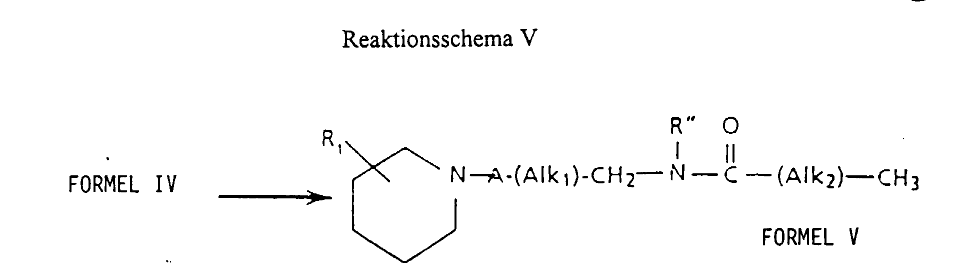
(E) für die Verbindungen, wiedergegeben durch Formel A, in der D -CH₂-N(R")-C(=NR"')- ist, E eine direkte Bindung darstellt, y 0 ist und die anderen Substituenten die gleichen Bedeutungen besitzen, wie zuerst in Formel A beschrieben wurde, Durchführung der im Reaktionsschema VI dargestellten Umsetzung durch (1) Alkylierung des Amids der Formel V mit einem Alkylierungsmittel, wie Triethyloxoniumtetrafluoroborat, und (2) Durchführung einer Austauschreaktion zwischen dem alkylierten Amid und einem Überschuß eines gewünschten Amins, wobei eine Verbindung der Formel VI hergestellt wird: 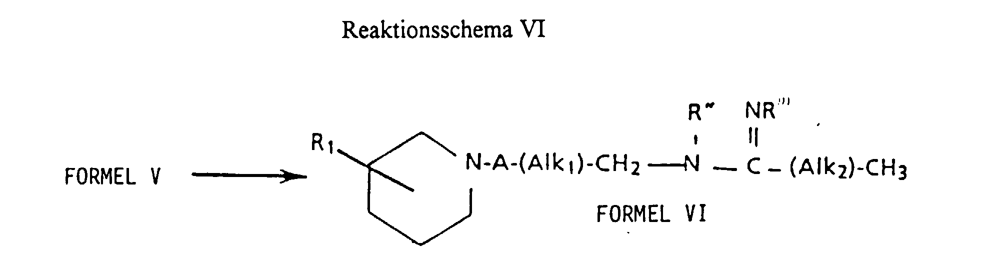
(F) für die Verbindungen, wiedergegeben durch Formel A, in der E -C(=NR"')-N(R")- ist und die anderen Substituenten die gleiche Bedeutung besitzen, wie zuerst in Fomel (A) beschrieben, Ausführung der im Reaktionsschema X dargestellten Umsetzung durch (1) Alkylierung des Amids der Formel IX mit einem Akylierungsmittel, wie Triethyloxoniumtetrafluoroborat, und (2) Durchführung einer Austauschreaktion zwischen dem alkylierten Amid und einem Überschuß des substituierten Amins H₂NR"', wobei R"' die gleiche Bedeutung besitzt, wie sie im Endprodukt gewünscht wird, wobei eine Verbindung der Formel X hergestellt wird:

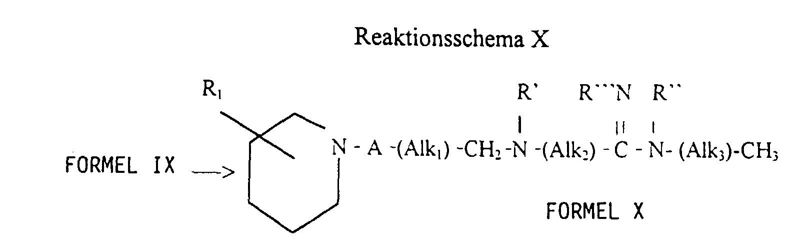

7. Verwendung einer Verbindung nach einem der Ansprüche 1 bis 5 zur Herstellung eines Arzneimittels zur Senkung des Cholesterinspiegels im Blut bei Säugern.

8. Verwendung einer Verbindung nach einem der Ansprüche 1 bis 5 zur Herstellung eines Arzneimittels zur Behandlung von Pilzinfektionen bei Tieren.

9. Arzneimittel, umfassend eine Verbindung nach einem der Ansprüche 1 bis 5, wobei die Verbindung in einer wirksamen Menge im Gemisch mit einem inerten Träger vorliegt.

10. Arzneimittel, umfassend eine Verbindung nach einem der Ansprüche 1 bis 5, wobei die Verbindung in einer wirksamen Menge im Gemisch mit einem pharmazeutisch verträglichen Träger vorliegt.

11. Arzneimittel nach Anspruch 9 oder 10 zur Senkung des Cholesterinspiegels im Blut von Säugern.

12. Arzneimittel nach Anspruch 9 oder 10 zur Behandlung von Pilzinfektionen bei Tieren.

## Patentansprüche (Patentansprüche für folgende(n) Vertragsstaat(en): ES)

1. Verfahren zur Herstellung einer Verbindung der Formel A: 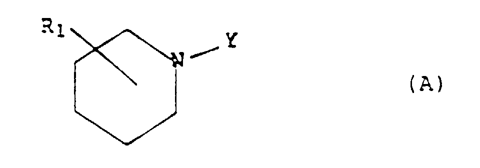 und der pharmazeutisch verträglichen Salze davon, wobei Y -A-(Alk₁)ₓ-D-(Alk₂)-E-(Alk₃)_{y}-CH₃ bedeutet, wobei der Rest A -CH₂- oder 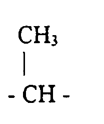 bedeutet;
D und E jeweils unabhängig 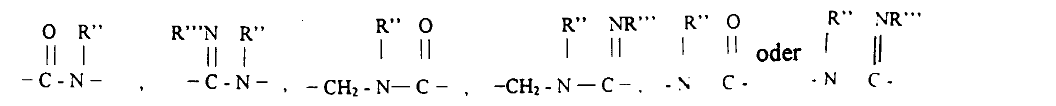 oder eine direkte Bindung bedeuten, mit der Maßgabe, daß wenn D einen Rest aus der Gruppe 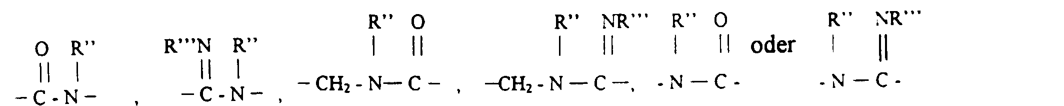 bedeutet, E nicht ein Rest aus der gleichen Gruppe sein kann; und daß D und E nicht beide direkte Bindungen sein können; (Alk₁), (Alk₂) und (Alk₃) bedeuten jeweils unabhängig einen geradkettigen Alkylenrest mit bis zu 5 Kohlenstoffatomen, der gegebenenfalls mit bis zu 3 Methylgruppen substituiert ist, oder (Alk₁), (Alk₂) und (Alk₃) bedeuten jeweils unabhängig einen geradkettigen Alkenylenrest mit 2 bis 6 Kohlenstoffatomen, wobei der geradkettige Alkenylenrest 1 bis 2 Doppelbindungen besitzt und gegebenenfalls mit bis zu 3 Methylgruppen substituiert ist; R₁ bedeutet ein Wasserstoffatom, eine Hydroxygruppe oder einen niederen C₁₋₄ Alkylrest;
R', R" und R"' bedeuten jeweils unabhängig ein Wasserstoffatom oder einen niederen C₁₋₄ Alkylrest; und x und y bedeuten unabhängig 0 oder 1; mit der Maßgabe, daß wenn A -CH₂- oder -CH(CH₃)- bedeutet und einer der Reste D oder E eine direkte Bindung ist, der andere der Reste D und E nicht CH₂-N(R')-, -N(R')- oder -C(O)N(R")- sein kann, ausgenommen wenn die Verbindung der Formel (A) N-(3-Methylbutyl)1-piperidinpropanamid, N-Butyl-N-methyl-1-piperidinbutyramid oder 4-(3-Methylbutylamino)-1-piperidinobutan und die pharmazeutisch verträglichen Salze davon darstellt, umfassend die Durchführung eines der nachstehenden Verfahrensschemata (A) bis (F):
(A) für die Verbindungen, wiedergegeben durch Formel A, in der D -C(O)N(R")- ist, A -CH₂-oder -CH(CH₃)- bedeutet, E eine direkte Bindung darstellt, y 0 ist und (Alk₁), (Alk₂), R" und x die vorstehenden Bedeutungen der Formel A besitzen, Durchführung der Umsetzung nach Reaktionsschema I durch (1) N-Alkylierung von Piperidin 2 durch einen Bromalkylester 1, wobei das Zwischenprodukt I hergestellt wird und (2) Durchführung einer Amidierungsreaktion zwischen dem Zwischenprodukt I und einem substituierten Amin 3, wobei eine Verbindung der Formel I hergestellt wird: 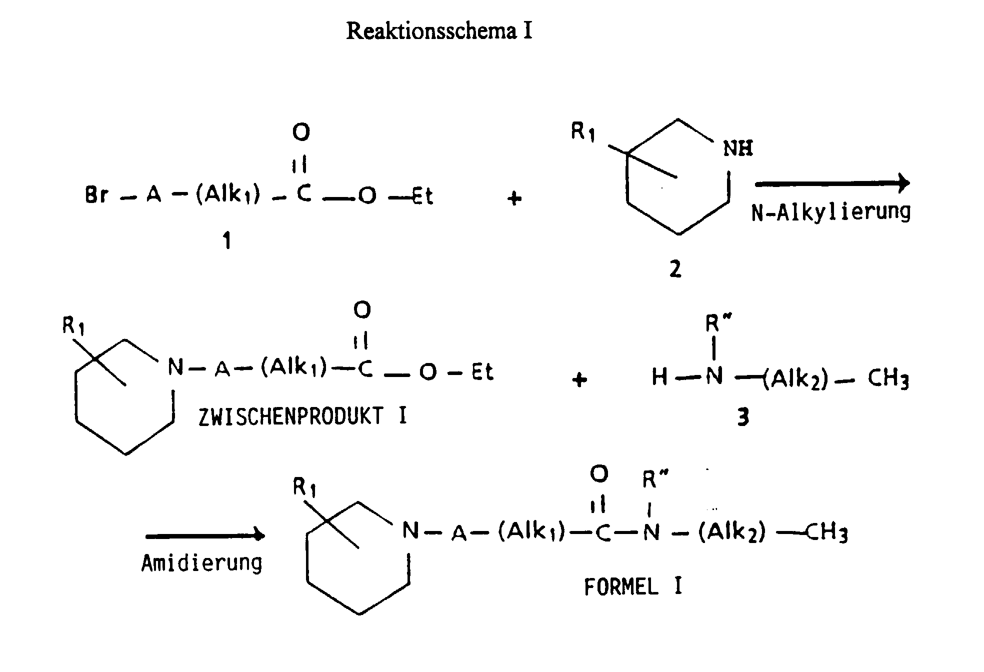
(B) für die Verbindungen, wiedergegeben durch Formel A, in der D -C(=NR"')-N(R")- ist, E eine direkte Bindung darstellt und (Alk₃) einen Alkylenrest mit 0 Kohlenstoffatomen bedeutet und die anderen Substituenten die gleichen Bedeutungen besitzen, wie zuerst in Formel A beschrieben, Durchführung der im Reaktionsschema III dargestellten Umsetzung durch (1) Alkylierung des Amids der Formel I mit einem Alkylierungsmittel, wie Triethyloxoniumtetrafluoroborat und (2) Durchführung einer Austauschreaktion zwischen dem alkylierten Amid und einem Überschuß des gewünschten Amins, wobei eine Verbindung der Formel III hergestellt wird: 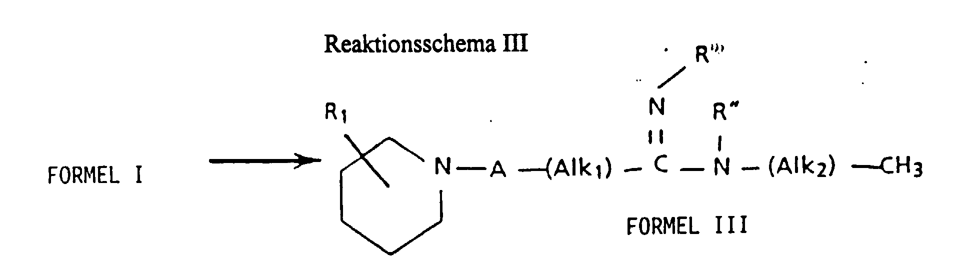
(C) für die Verbindungen, wiedergegeben durch Formel A, wobei D -CH₂-N(R")-C(=NR"')- bedeutet, R"' ein Wasserstoffatom bedeutet, der Rest A -CH₂- oder -CH(CH₃)- bedeutet, E eine direkte Bindung darstellt, y 0 ist und (Alk₁), (Alk₂), R₁, R" und x die gleiche Bedeutung besitzen, wie zuerst in Formel A beschrieben wurde, Durchführung der im Reaktionsschema IV beschriebenen Umsetzung durch (1) eine Pinner-Reaktion, wobei das Alkylnitril 1, wobei (Alk₂) die gleiche Bedeutung besitzt, wie sie im Endprodukt gewünscht wird, zum Imidatester 3 umgewandelt wird und (2) Durchführung einer Amidierungsreaktion zwischen dem Imidatester 3 und dem Zwischenprodukt II, wobei eine Verbindung der Formel IV hergestellt wird: 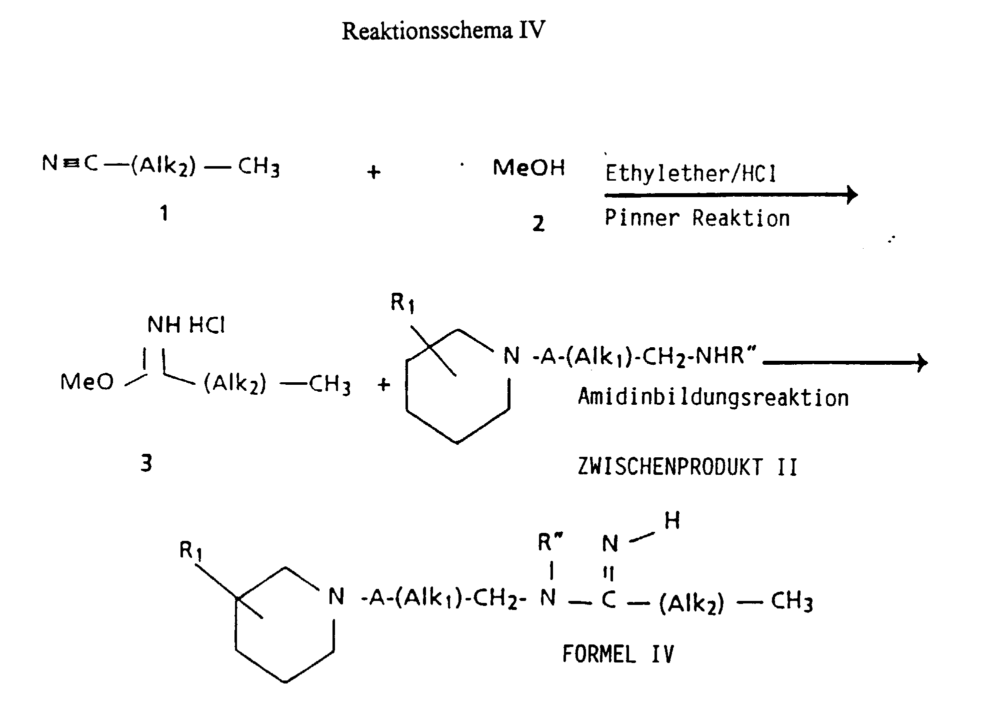 wodurch das Zwischenprodukt II erhältlich ist, in dem R" ein Wasserstoffatom bedeutet (Zwischenprodukt IIA), durch Reduktion des Alkylcyanopiperidins IA, in dem A und Alk₁ die gleiche Bedeutung besitzen, wie sie für das Endprodukt gewünscht werden, mit einem Reduktionsmittel, wie Lithiumaluminiumhydrid: 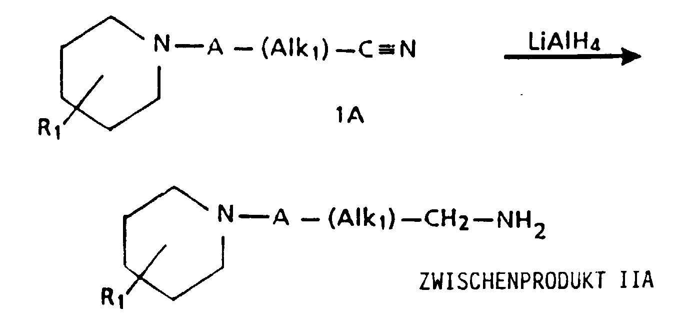 und wobei das Zwischenprodukt II, wobei R" einen niederen C₁-C₄ Alkylrest (Zwischenprodukt IIB) bedeutet, wie nachstehend wiedergegeben erhältlich ist: 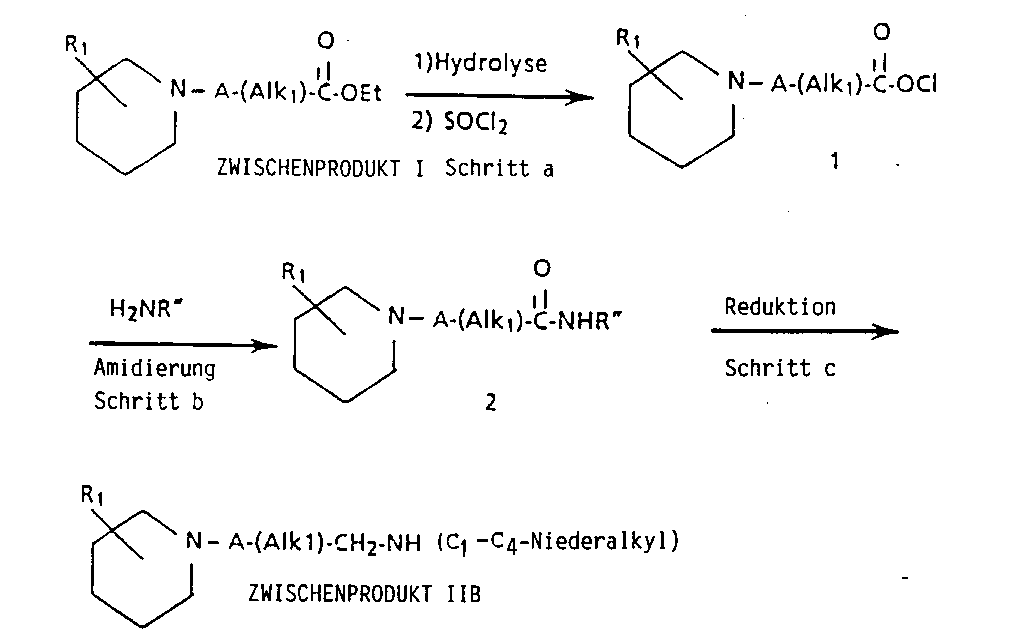
(D) für die Verbindungen, wiedergegeben durch Formel A, in der D -CH₂-N(R")-C(O)- ist, E eine direkte Bindung darstellt, y 0 ist und die anderen Substituenten die gleiche Bedeutung besitzen, wie sie zuerst in Formel A beschrieben sind, Durchführung der im Reaktionsschema V dargestellten Umsetzung durch saure Hydrolyse des Amidins der Formel IV, wobei das Amid der Formel V hergestellt wird: 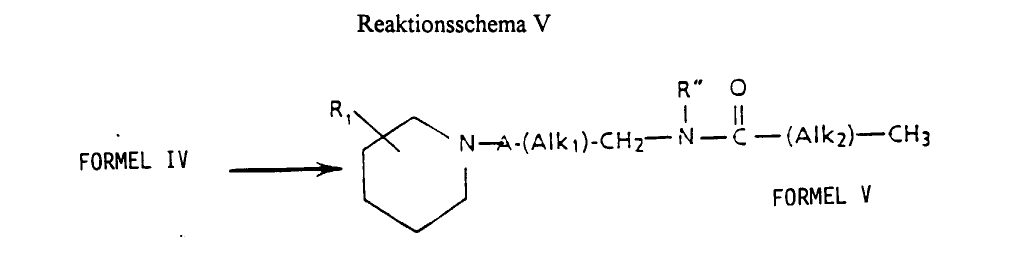
(E) für die Verbindungen, wiedergegeben durch Formel A, in der D -CH₂-N(R")-C(=NR"')- ist, E eine direkte Bindung darstellt, Y 0 ist und die anderen Substituenten die gleichen Bedeutungen besitzen, wie zuerst in Formel A beschrieben wurde, Durchführung der im Reaktionsschema VI dargestellten Umsetzung durch (1) Alkylierung des Amids der Formel V mit einem Alkylierungsmittel, wie Triethyloxoniumtetrafluoroborat, und (2) Durchführung einer Austauschreaktion zwischen dem alkylierten Amid und einem Überschuß eines gewünschten Amins, wobei eine Verbindung der Formel VI hergestellt wird: 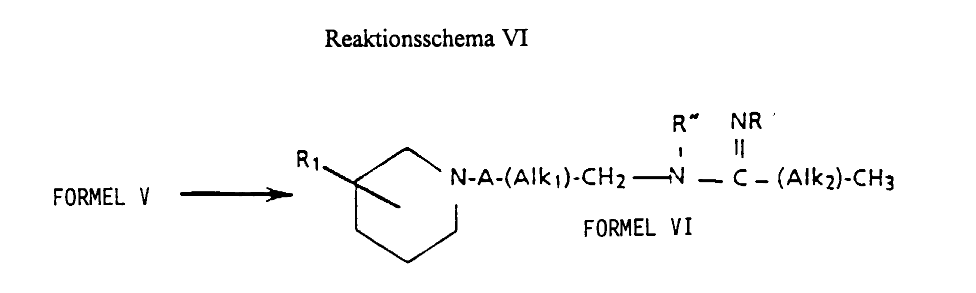
(F) für die Verbindungen, wiedergegeben durch Formel A, in der E -C(=NR"')-N(R")- ist und die anderen Substituenten die gleiche Bedeutung besitzen, wie zuerst in Fomel (A) beschrieben, Ausführung der im Reaktionsschema X dargestellten Umsetzung durch (1) Alkylierung des Amids der Formel IX mit einem Akylierungsmittel, wie Triethyloxoniumtetrafluoroborat, und (2) Durchführung einer Austauschreaktion zwischen dem alkylierten Amid und einem Überschuß des substituierten Amins H₂NR"', wobei R"' die gleiche Bedeutung besitzt, wie sie im Endprodukt gewünscht wird, wobei eine Verbindung der Formel X hergestellt wird:

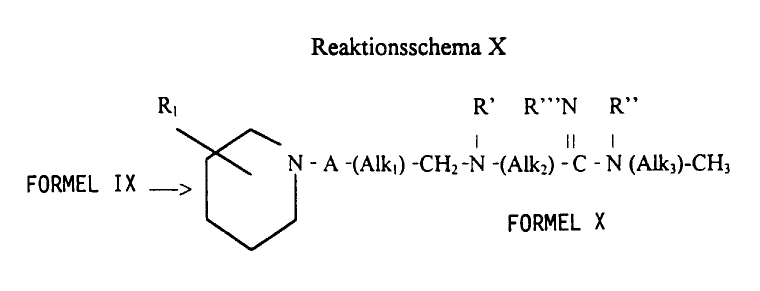

2. Verfahren zur Herstellung von N-(3-Piperidinopropyl)-4-methylvalerylamidin nach Anspruch 1 und der pharmazeutisch verträglichen Salze davon.

3. Verfahren zur Herstellung von N-(4-Piperidinobutyl)-4-methylvalerylamidin nach Anspruch 1 und der pharmazeutisch verträglichen Salze davon.

4. Verfahren zur Herstellung von Piperidinooctylamidin nach Anspruch 1 und der pharmazeutisch verträglichen Salze davon.

5. Verfahren zur Herstellung von N-(1-Trifluormethylundecan)-piperidin nach Anspruch 1.

## Patentansprüche (Patentansprüche für folgende(n) Vertragsstaat(en): GR)

1. Verbindung, wiedergegeben durch Formel A: 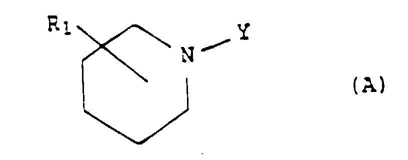 und der pharmazeutisch verträglichen Salze davon, wobei Y -A-(Alk₁)ₓ-D-(Alk₂)-E-(Alk₃)_{y}-CH₃ bedeutet, wobei der Rest A -CH₂- oder 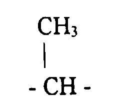 bedeutet;
D und E jeweils unabhängig 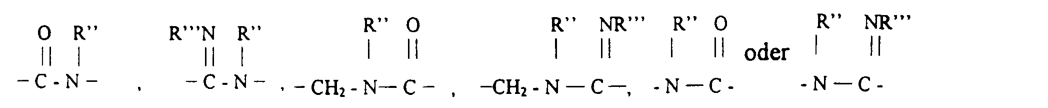 oder eine direkte Bindung bedeuten, mit der Maßgabe, daß wenn D einen Rest aus der Gruppe 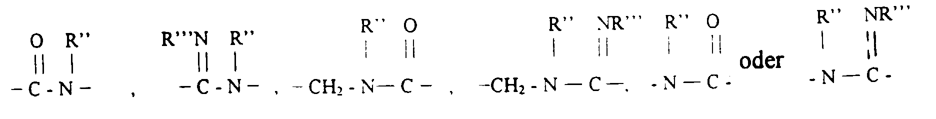 bedeutet, E nicht ein Rest aus der gleichen Gruppe sein kann; und daß D und E nicht beide direkte Bindungen sein können; (Alk₁), (Alk₂) und (Alk₃) bedeuten jeweils unabhängig einen geradkettigen Alkylenrest mit bis zu 5 Kohlenstoffatomen, der gegebenenfalls mit bis zu 3 Methylgruppen substituiert ist, oder (Alk₁), (Alk₂) und (Alk₃) bedeuten jeweils unabhängig einen geradkettigen Alkenylenrest mit 2 bis 6 Kohlenstoffatomen, wobei der geradkettige Alkenylenrest 1 bis 2 Doppelbindungen besitzt und gegebenenfalls mit bis zu 3 Methylgruppen substituiert ist; R₁ bedeutet ein Wasserstoffatom, eine Hydroxygruppe oder einen niederen C₁₋₄ Alkylrest;
R', R" und R"' bedeuten jeweils unabhängig ein Wasserstoffatom oder einen niederen C₁₋₄ Alkylrest; und x und y bedeuten unabhängig 0 oder 1; mit der Maßgabe, daß wenn A -CH₂- oder -CH(CH₃)- bedeutet und einer der Reste D oder E eine direkte Bindung ist, der andere der Reste D und E nicht CH₂-N(R')-, -N(R')- oder -C(O)N(R")- sein kann, ausgenommen wenn die Verbindung der Formel (A) N-(3-Methylbutyl)1-piperidinpropanamid, N-Butyl-N-methyl-1-piperidinbutyramid oder 4-(3-Methylbutylamino)-1-piperidinobutan und die pharmazeutisch verträglichen Salze davon darstellt.

2. N-(3-Piperidinopropyl)-4-methylvalerylamidin nach Anspruch 1 und die pharmazeutisch verträglichen Salze davon.

3. N-(4-Piperidinobutyl)-4-methylvalerylamidin nach Anspruch 1 und die pharmazeutisch verträglichen Salze davon.

4. Piperidinoctylamidin nach Anspruch 1 und die pharmazeutisch verträglichen Salze davon.

5. N-(1-Trifluormethylundecan)-piperidin nach Anspruch 1.

6. Verfahren zur Herstellung einer Verbindung nach einem der Ansprüche 1 bis 5, umfassend die Durchführung eines der nachstehenden Verfahrensschemata (A) bis (F):
(A) für die Verbindungen, wiedergegeben durch Formel A, in der D -C(O)N(R")- ist, A -CH₂- oder -CH(CH₃)- bedeutet, E eine direkte Bindung darstellt, y 0 ist und (Alk₁), (Alk₂), R" und x die vorstehenden Bedeutungen der Formel A besitzen, Durchführung der Umsetzung nach Reaktionsschema I durch (1) N-Alkylierung von Piperidin 2 durch einen Bromalkylester 1, wobei das Zwischenprodukt I hergestellt wird und (2) Durchführung einer Amidierungsreaktion zwischen dem Zwischenprodukt I und einem substituierten Amin 3, wobei eine Verbindung der Formel I hergestellt wird: 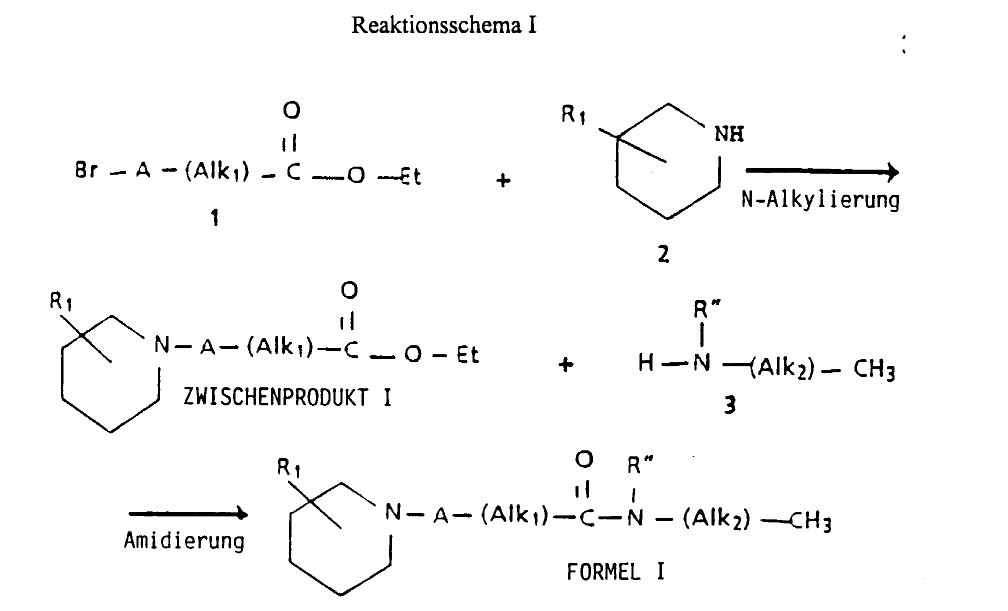
(B) für die Verbindungen, wiedergegeben durch Formel A, in der D -C(=NR"')-N(R")- ist, E eine direkte Bindung darstellt und (Alk₃) einen Alkylenrest mit 0 Kohlenstoffatomen bedeutet und die anderen Substituenten die gleichen Bedeutungen besitzen, wie zuerst in Formel A beschrieben, Durchführung der im Reaktionsschema III dargestellten Umsetzung durch (1) Alkylierung des Amids der Formel I mit einem Alkylierungsmittel, wie Triethyloxoniumtetrafluoroborat und (2) Durchführung einer Austauschreaktion zwischen dem alkylierten Amid und einem Überschuß des gewünschten Amins, wobei eine Verbindung der Formel III hergestellt wird: 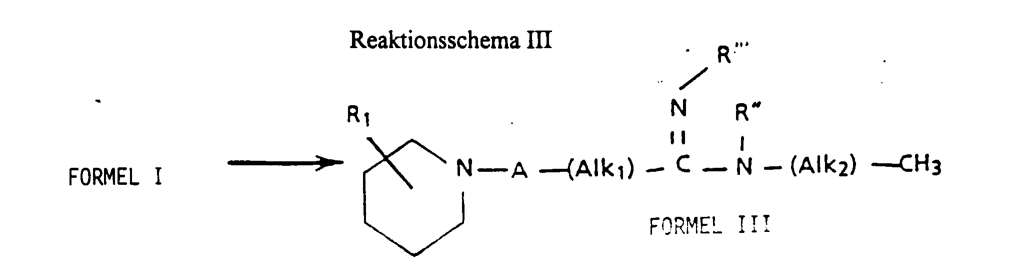
(C) für die Verbindungen, wiedergegeben durch Formel A, wobei D -CH₂-N(R")-C(=NR"')- bedeutet, R"' ein Wasserstoffatom bedeutet, der Rest A -CH₂- oder -CH(CH₃)- bedeutet, E eine direkte Bindung darstellt, y 0 ist und (Alk₁), (Alk₂), R₁, R" und x die gleiche Bedeutung besitzen, wie zuerst in Formel A beschrieben wurde, Durchführung der im Reaktionsschema IV beschriebenen Umsetzung durch (1) eine Pinner-Reaktion, wobei das Alkylnitril 1, wobei (Alk₂) die gleiche Bedeutung besitzt, wie sie im Endprodukt gewünscht wird, zum Imidatester 3 umgewandelt wird und (2) Durchführung einer Amidierungsreaktion zwischen dem Imidatester 3 und dem Zwischenprodukt II, wobei eine Verbindung der Formel IV hergestellt wird: 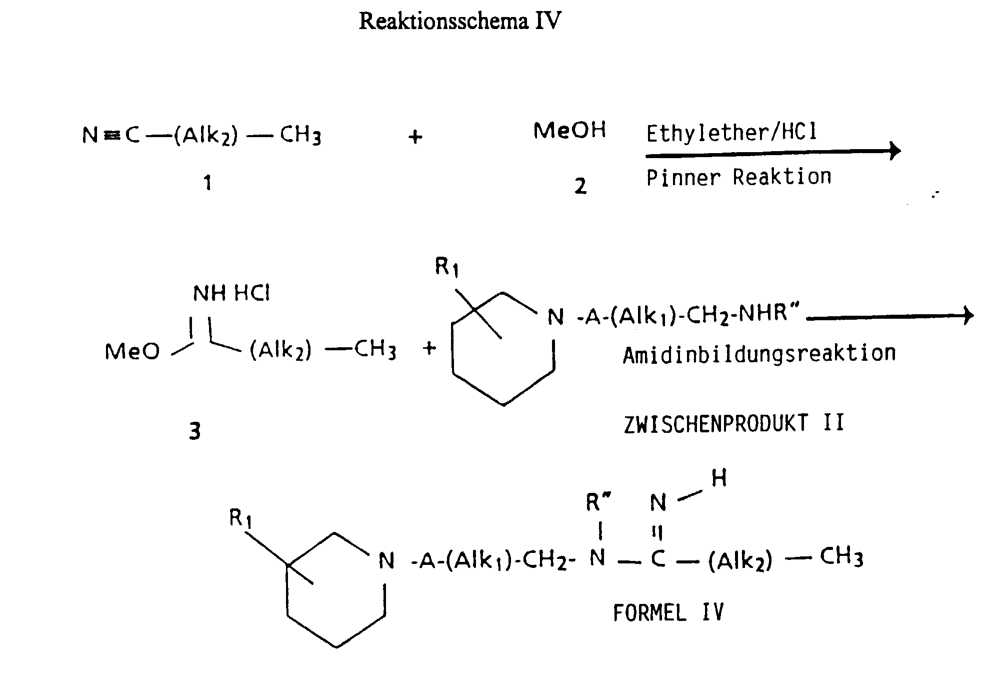 wodurch das Zwischenprodukt II erhältlich ist, in dem R" ein Wasserstoffatom bedeutet (Zwischenprodukt IIA), durch Reduktion des Alkylcyanopiperidins IA, in dem A und Alk₁ die gleiche Bedeutung besitzen, wie sie für das Endprodukt gewünscht werden, mit einem Reduktionsmittel, wie Lithiumaluminiumhydrid: 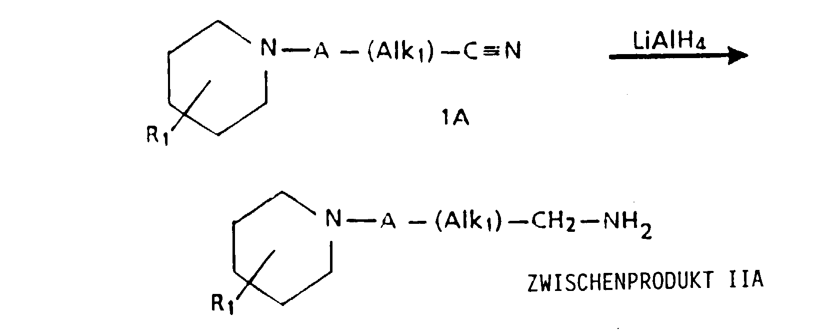 und wobei das Zwischenprodukt II, wobei R" einen niederen C₁-C₄ Alkylrest (Zwischenprodukt IIB) bedeutet, wie nachstehend wiedergegeben erhältlich ist: 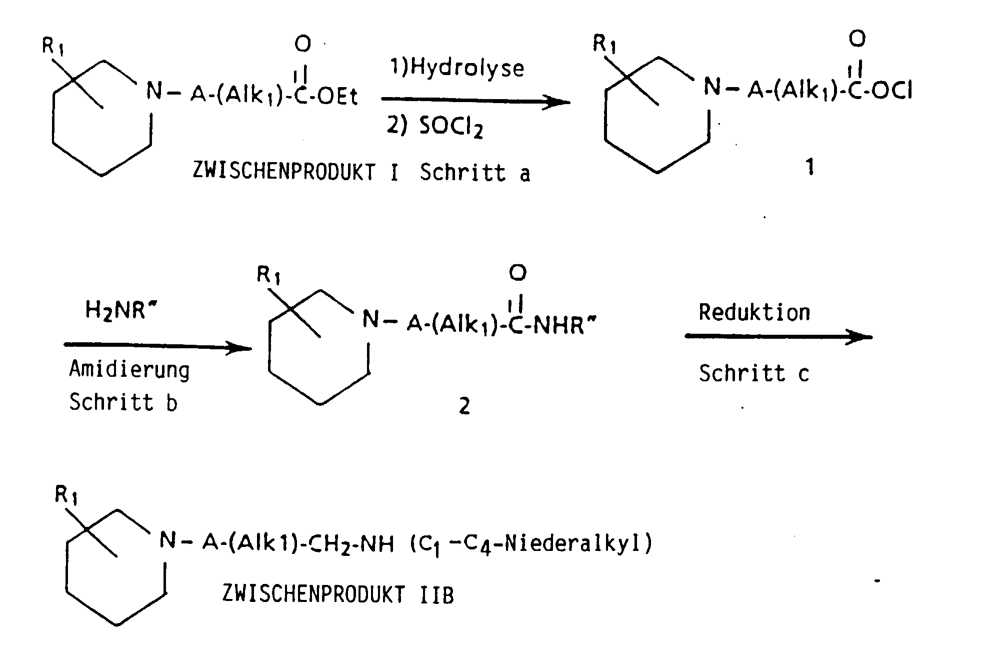
(D) für die Verbindungen, wiedergegeben durch Formel A, in der D -CH₂-N(R")-C(O)- ist, E eine direkte Bindung darstellt, y 0 ist und die anderen Substituenten die gleiche Bedeutung besitzen, wie sie zuerst in Formel A beschrieben sind, Durchführung der im Reaktionsschema V dargestellten Umsetzung durch saure Hydrolyse des Amidins der Formel IV, wobei das Amid der Formel V hergestellt wird: 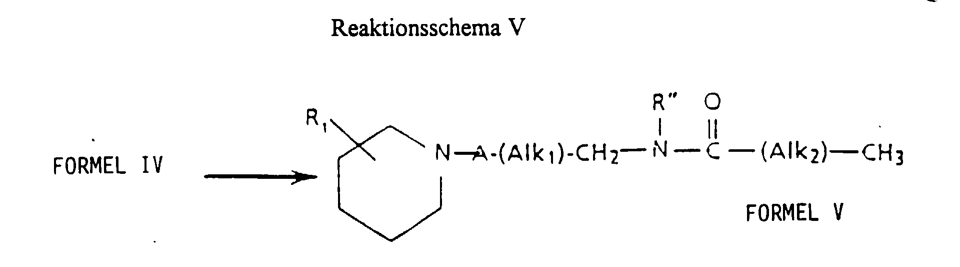
(E) für die Verbindungen, wiedergegeben durch Formel A, in der D -CH₂-N(R")-C(=NR"')- ist, E eine direkte Bindung darstellt, y 0 ist und die anderen Substituenten die gleichen Bedeutungen besitzen, wie zuerst in Formel A beschrieben wurde, Durchführung der im Reaktionsschema VI dargestellten Umsetzung durch (1) Alkylierung des Amids der Formel V mit einem Alkylierungsmittel, wie Triethyloxoniumtetrafluoroborat, und (2) Durchführung einer Austauschreaktion zwischen dem alkylierten Amid und einem Überschuß eines gewünschten Amins, wobei eine Verbindung der Formel VI hergestellt wird: 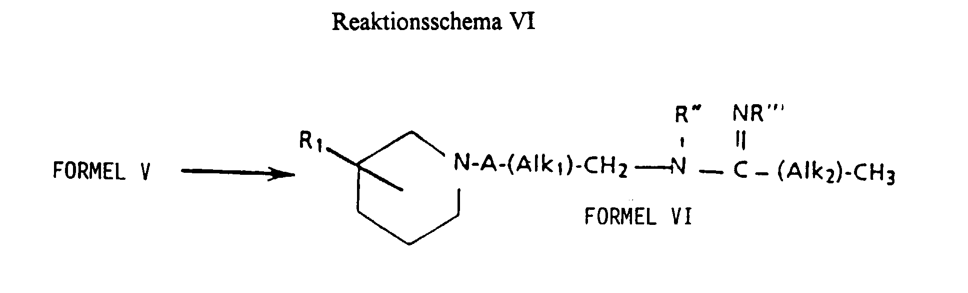
(F) für die Verbindungen, wiedergegeben durch Formel A, in der E -C(=NR"')-N(R")- ist und die anderen Substituenten die gleiche Bedeutung besitzen, wie zuerst in Fomel (A) beschrieben, Ausführung der im Reaktionsschema X dargestellten Umsetzung durch (1) Alkylierung des Amids der Formel IX mit einem Akylierungsmittel, wie Triethyloxoniumtetrafluoroborat, und (2) Durchführung einer Austauschreaktion zwischen dem alkylierten Amid und einem Überschuß des substituierten Amins H₂NR"', wobei R"' die gleiche Bedeutung besitzt, wie sie im Endprodukt gewünscht wird, wobei eine Verbindung der Formel X hergestellt wird:

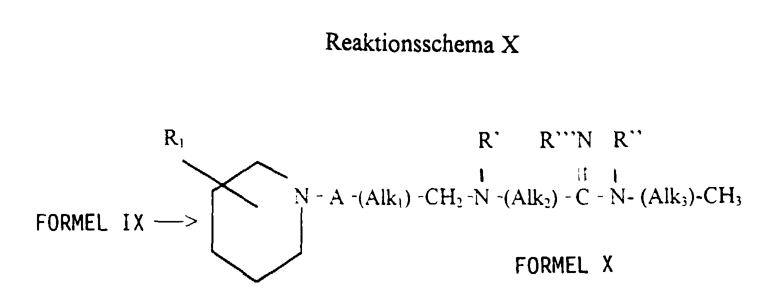

7. Verwendung einer Verbindung nach einem der Ansprüche 1 bis 5 zur Herstellung eines Arzneimittels zur Senkung des Cholesterinspiegels im Blut bei Säugern.

8. Verwendung einer Verbindung nach einem der Ansprüche 1 bis 5 zur Herstellung eines Arzneimittels zur Behandlung von Pilzinfektionen bei Tieren.

## Revendications (Revendications pour l'(les) Etat(s) contractant(s) suivant(s): AT, BE, CH, DE, DK, FR, GB, IT, LI, LU, NL, SE)

1. Composés selon la formule (A): 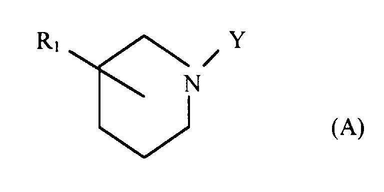 et ses sels acceptables du point de vue pharmaceutique, dans laquelle
Y représente -A-(Alk₁)ₓ-D-(Alk₂)-E-(Alk₃)_{y}-CH₃, dans lequel le fragment A représente -CH₂- ou 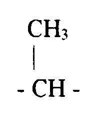 D et E représentent chacun indépendamment 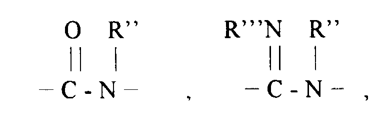 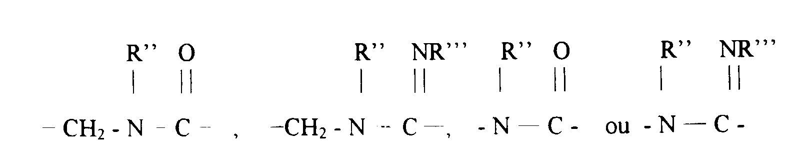 ou une liaison directe, à la condition que lorsque D représente un fragment choisi dans le groupe 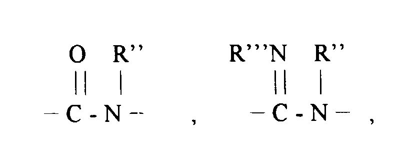 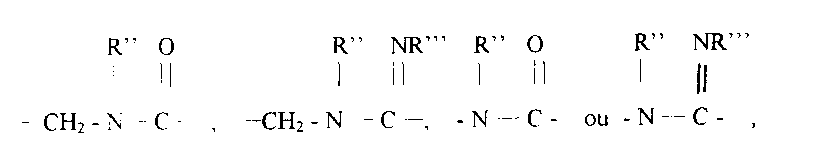 E ne puisse pas représenter un fragment du même groupe, et que D et E ne puissent pas être tous deux des liaisons directes;
(Alk₁), (Alk₂) et (Alk₃) représentent chacun indépendamment un fragment alkyléne à chaîne linéaire contenant jusqu'à 5 atomes de carbone, éventuellement substitué par jusqu'à 3 groupes méthyle, ou
(Alk₁), (Alk₂) et (Alk₃) représentent chacun indépendamment un fragment alcénylène à chaîne linéaire contenant de 2 à 6 atomes de carbone, le fragment alcénylène à chaîne linéaire comportant de 1 à 2 double liaisons, et étant éventuellement substitué par jusqu'à 3 groupes méthyle;
R₁ représente l'hydrogène, un groupe hydroxy ou alkyle inférieur en C₁₋₄; R', R", R"' représentent chacun indépendamment l'hydrogène ou un groupe alkyle inférieur en C₁₋₄ ;
et x et y représentent chacun indépendamment 0 ou 1; à la condition que quand A
est - CH₂ - ou - CH (CH₃) - et l'un de D ou E est une liaison directe, l'autre de D ou E n'est pas - C (O) N (R") - excepté quand le composé de formule (A) est le N- (3-méthylbutyl)1-pipéridine propanamide,
le N-butyl-N-méthyl-1-pipéridinebutyramide ou le 4-(3-méthylbutylamino)-1-pipéridinobutane et ses sels acceptables du point de vue pharmaceutique.

2. Composé selon la revendication 1, caractérisé en ce qu'il s'agit de la N-(3- pipéridinopropyl)-4-méthylvalérylamidine, et ses sels acceptables du point de vue pharmaceutique.

3. Composé selon la revendication 1, caractérisé en ce qu'il s'agit de la N-(4-pipéridinobutyl)-4-méthylvalérylamidine, et ses sels acceptables du point de vue pharmaceutique.

4. Composé selon la revendication 1, caractérisé en ce qu'il s'agit de la pipéridine octylamidine, et ses sels acceptables du point de vue pharmaceutique.

5. Composé selon la revendication 1, caractérisé en ce qu'il s'agit de la N-(1-trifluorométhyl-undécane)-pipéridine.

6. Procédé de préparation d'un composé selon l'une des revendications 1 à 5, caractérisé en ce qu'il comprend une des étapes de procédé suivante (A) à (F):
(A) pour ceux des composés de formule A dans laquelle D est - C (O) - N(R") - , A est -CH₂ - ou - CH (CH₃) -, E est une liaison directe, y est égal à 0 et (Alk₁), (Alk₂), R" et x sont tels que définis en premier lieu dans la Formule A, en effectuant la réaction décrite dans le schéma réactionnel I en (1) réalisant la N-alkylation de la pipéridine 2 par un ester bromoalkylique 1, pour produire l'intermédiaire I et (2) en réalisant une réaction d'amidation entre l'Intermédiaire I et une amine substituée 3 pour produire un composé de Formule I: 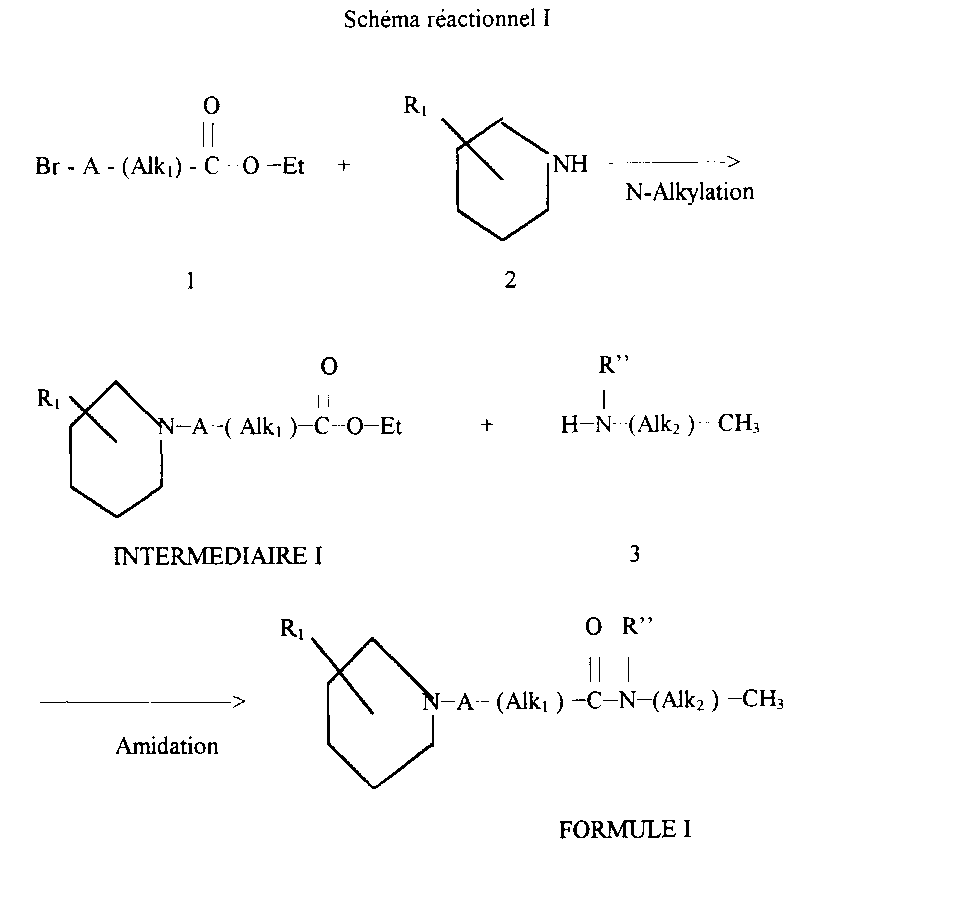
(B) pour ceux des composés selon la Formule A dans laquelle D est -C(=NR"')-N(R")-, E est une liaison directe et (Alk₃) est un fragment alkyléne à 0 atomes de carbone et les autres substituants sont tels que définis en premier lieu dans la Formule A, en effectuant la réaction décrite dans le schéma réactionnel III en (1) réalisant une alkylation de l'amide de Formule I avec un agent d'alkylation, tel que le tétrafluoroborate de triéthyloxonium et (2) en réalisant une réaction de déplacement entre l'amide alkylé et une quantité en excès de l'amine désirée pour produire un composé de Formule III: 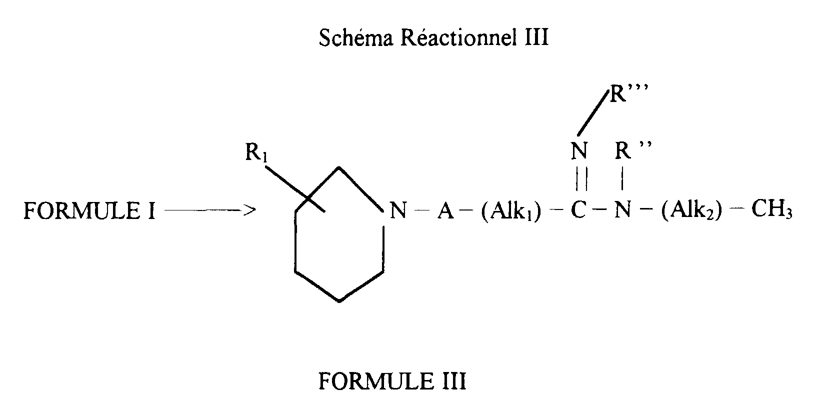
( C) pour ceux des composés selon la Formule A dans laquelle D est -CH₂-N-(R")-C(=NR"')-,
R"' est l'hydrogène, le fragment A est -CH₂- ou -CH(CH₃)-, E est une liaison directe, y est égal à 0 et (Alk₁), (Alk₂), R1, R" et x sont tels que définis en premier lieu dans la Formule A, en effectuant la réaction décrite dans le Schéma Réactionnel IV en
(1) réalisant une réaction Pinner selon laquelle l'alkylnitrile 1, dans lequel Alk₂ a la même définition que celle désirée dans le produit final, est converti en ester amidate 3 et
(2) réalisant une réaction d'amidation entre ester amidate 3 et l'Intermédiaire II pour produire un composé de Formule IV: 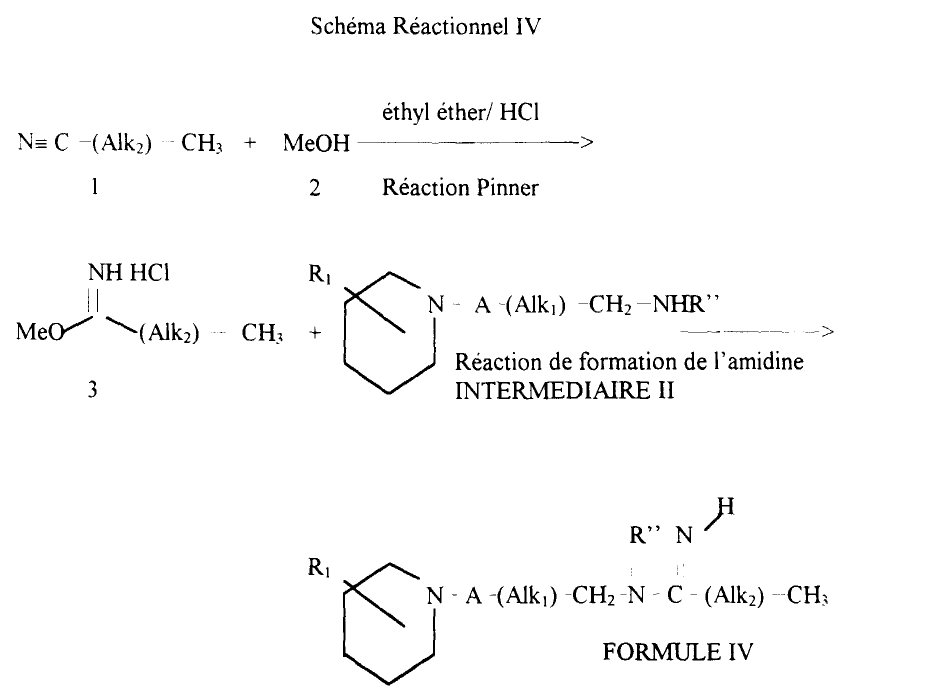
selon lequel on obtient l'Intermédiaire II, dans lequel R " est l'hydrogène (Intermédiaire IIA), en réalisant une réduction de l'alkylcyano pipéridine IA, dans laquelle A et Alk₁ ont les mêmes définitions que celles désirées dans le produit final, avec un agent réducteur, tel que l'hydrure de lithium aluminium: 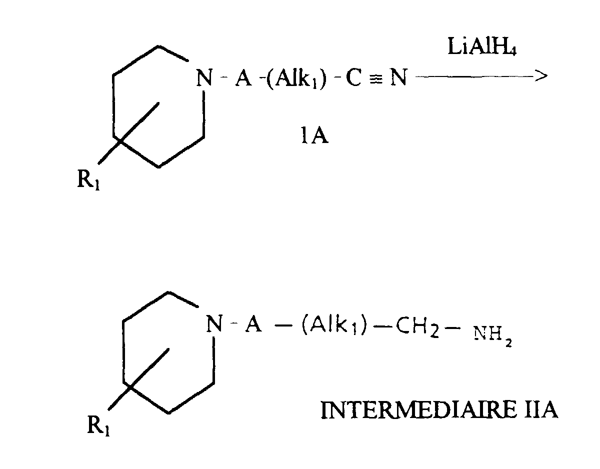 et selon lequel on obtient l'Intermédiaire II. dans lequel R" est un alkyle inférieur en C₁-C₄ ( Intermédiaire IIB), de la manière suivante: 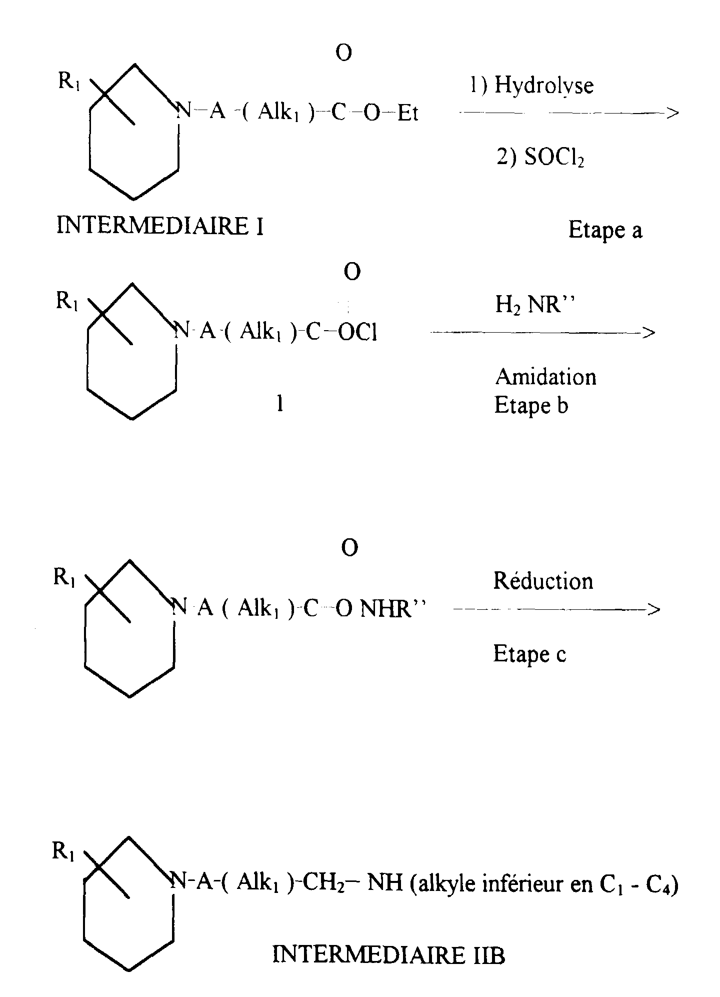
(D) pour ceux des composés selon la Formule A dans laquelle D est -CH₂-N(R")-C(O)- , E est une liaison directe, y est égal à 0 et les autres substituants sont tels que définis en premier lieu dans la Formule A, en effectuant la réaction décrite dans le schéma réactionnel V en réalisant une hydrolyse acide de l'amidine de Formule IV pour produire l'amide de Formule V: 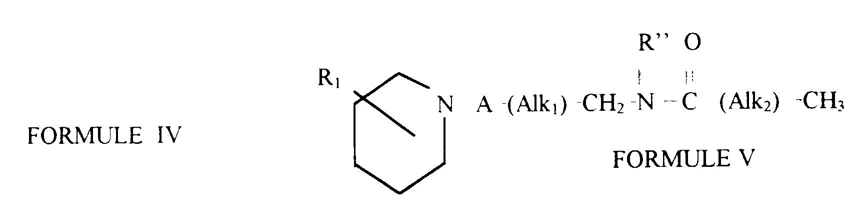
(E) pour ceux des composés selon la Formule A dans laquelle D est -CH₂-N(R")-C(=NR"')-, E est une liaison directe, y est égal à 0 et les autres substituants sont tels que définis en premier lieu dans la Formule A, en effectuant la réaction décrite dans le Schéma Réactionnel VI en (1) réalisant une alkylation de l'amide de Formule V avec un agent d'alkylation, tel que le tétrafluoroborate de triéthyloxonium et (2) en réalisant une réaction de déplacement entre l'amide alkylé et un excès de l'amine désirée pour produire un composé de Formule VI: 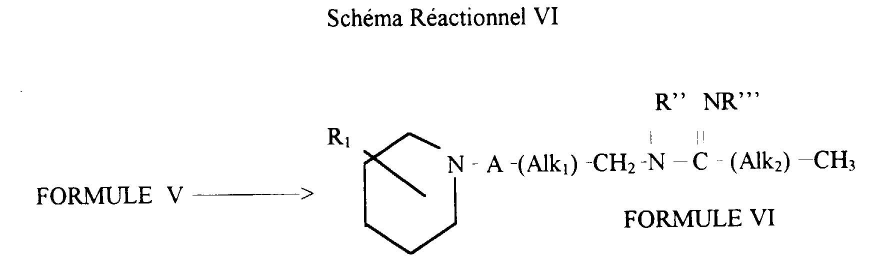
(F) pour ceux des composés selon la Formule A dans laquelle E est -C(=NR"')- N(R")-et les autres substituants sont tels que définis en premier lieu dans la Formule (A), en effectuant la réaction décrite dans le Schéma réactionnel X en
(1) réalisant une alkylation de l'amide de Formule IX avec un agent d'alkylation, tel que le tétrafluoroborate de triéthyloxonium et
(2) en réalisant une réaction de déplacement entre l'amide alkylé et un excès d'amine substituée H₂NR"', dans laquelle R"' a la même signification que dans le produit final désiré, pour produire le composé de la Formule X:

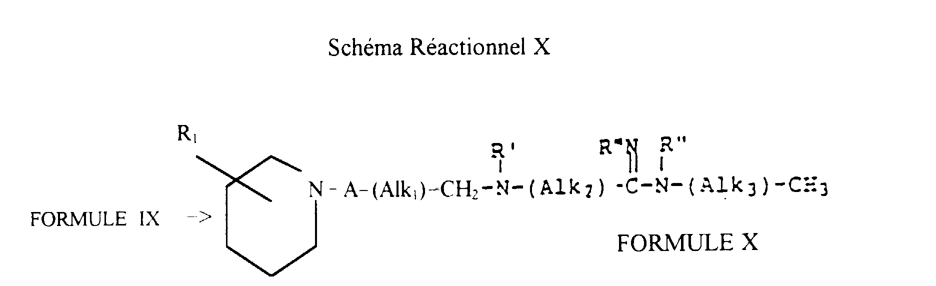

7. Utilisation d'un composé selon l'une des revendications 1 à 5 pour la préparation d'une composition pharmaceutique pour l'abaissement du cholestérol dans le plasma chez les mammifères.

8. Utilisation d'un composé selon l'une des revendications 1 à 5 pour la préparation d'une composition pharmaceutique pour le traitement d'infections fongiques chez les animaux

9. Composition pharmaceutique comprenant un composé selon l'une des revendications 1 à 5, présent en une quantité active en mélange avec un support inerte.

10. Composition pharmaceutique comprenant un composé selon l'une des revendications 1 à 5, présent en une quantité active en mélange avec un support acceptable du point de vue pharmaceutique.

11. Composition pharmaceutique selon l'une des revendications 9 ou 10 pour l'abaissement du cholestérol dans le plasma chez les mammifères.

12. . Composition pharmaceutique selon l'une des revendications 9 ou 10 pour le traitement d'infections fongiques chez les animaux.

## Revendications (Revendications pour l'(les) Etat(s) contractant(s) suivant(s): ES)

1. Procédé de préparation d'un composé selon la formule (A): 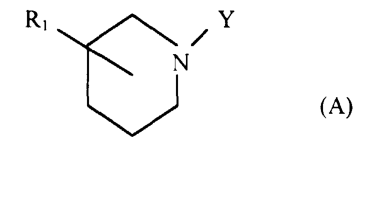 et ses sels acceptables du point de vue pharmaceutique, dans laquelle
Y représente -A-(Alk₁)ₓ-D-(Alk₂)-E-(Alk₃)_{y}-CH₃, dans lequel le fragment A représente -CH₂- ou 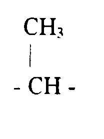 D et E représentent chacun indépendamment 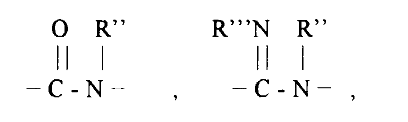 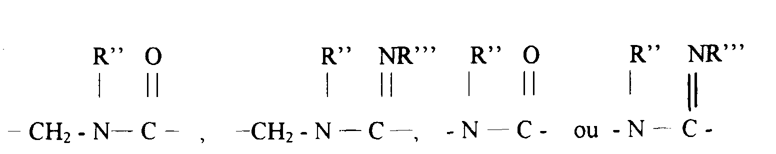 ou une liaison directe, à la condition que lorsque D représente un fragment choisi dans le groupe 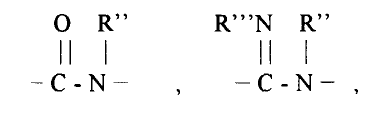 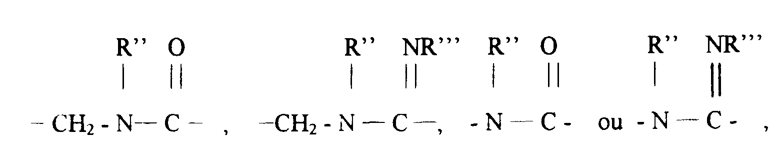 E ne puisse pas représenter un fragment du même groupe, et que D et E ne puissent pas être tous deux des liaisons directes;
(Alk₁), (Alk₂) et (Alk₃) représentent chacun indépendamment un fragment alkylène à chaîne linéaire contenant jusqu'à 5 atomes de carbone, éventuellement substitué par jusqu'à 3 groupes méthyle, ou
(Alk₁), (Alk₂) et (Alk₃) représentent chacun indépendamment un fragment alcénylène à chaîne linéaire contenant de 2 à 6 atomes de carbone, le fragment alcénylène à chaîne linéaire comportant de 1 à 2 double liaisons, et étant éventuellement substitué par jusqu'à 3 groupes méthyle;
R₁ représente l'hydrogène, un groupe hydroxy ou alkyle inférieur en C₁₋₄ ; R', R", R"' représentent chacun indépendamment l'hydrogène ou un groupe alkyle inférieur en C₁₋₄ ;
et x et y représentent chacun indépendamment 0 ou 1; à la condition que quand A est -CH₂- ou -CH(CH3)- et l'un de D our E est une liaison directe, l'autre de D ou E n'est pas -CH₂-N(R')-, -N(R')- ou -C(O)N(R")- excepté quand le composé de formula (A) est
le N-(3-méthylbutyl)1-pipéridine propanamide,
le N-butyl-N-méthyl-1-pipéridinebutyramide ou
le 4-(3-méthylbutylamino)-1-pipéridinobutane et ses sels acceptables du point de vue pharmaceutique; comprenant la réalisation d'une des étapes de procédé suivantes (A) à (F):
(A) pour ceux des composés de formule A dans laquelle D est -C(O)-N(R")- , A est -CH₂ - ou - CH (CH₃) -, E est une liaison directe, y est égal à 0 et (Alk₁), (Alk₂), R" et x sont tels que définis en premier lieu dans la Formule A, en effectuant la réaction décrite dans le schéma réactionnel I en (1) réalisant la N-alkylation de la pipéridine 2 par un ester bromoalkylique 1, pour produire l'Intermédiaire I et (2) en réalisant une réaction d'amidation entre l'intermédiaire I et une amine substituée 3 pour produire la Formule I: 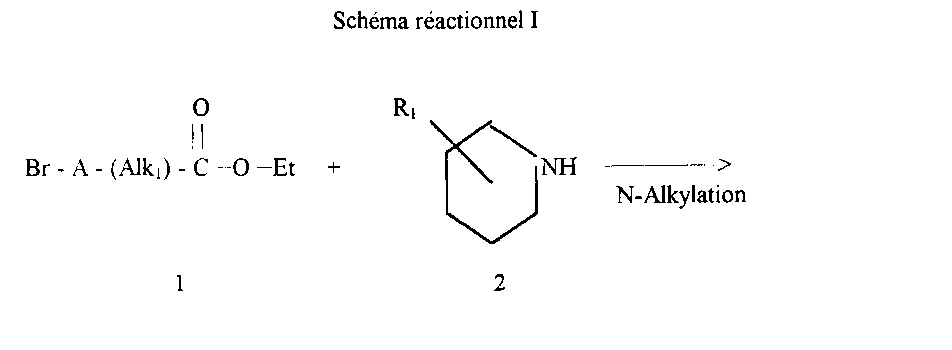 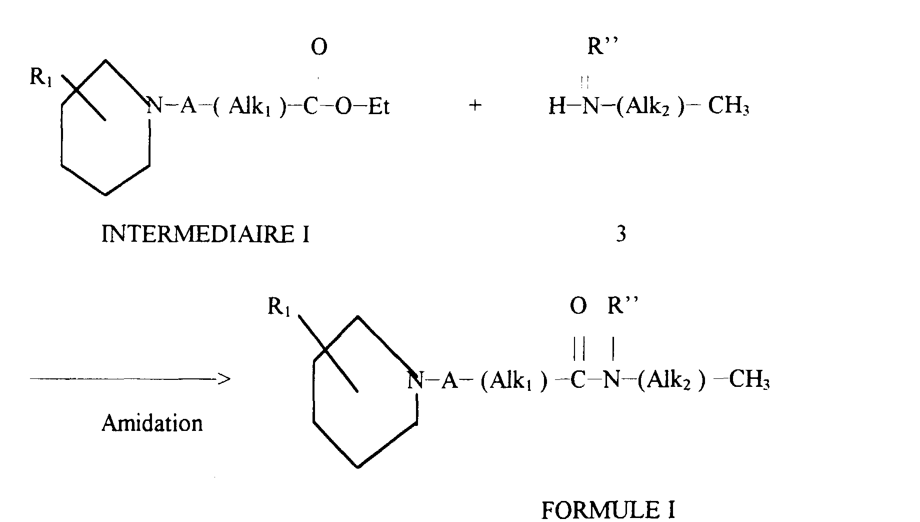
(B) pour ceux des composés selon la Formule A dans laquelle D est -C(=NR"')-N( R")-, E est une liaison directe et (Alk₃) est un fragment alkylène à 0 atomes de carbone et les autres substituants sont tels que définis en premier lieu dans le Formule A, en effectuant la réaction décrite dans le schéma réactionnel III en (1) réalisant une alkylation de l'amide de Formule I avec un agent d'alkylation, tel que le tétrafluoroborate de triéthyloxonium et (2) en réalisant une réaction de déplacement entre l'amide alkylé et une quantité en excès de l'amine désirée pour produire la Formule III: 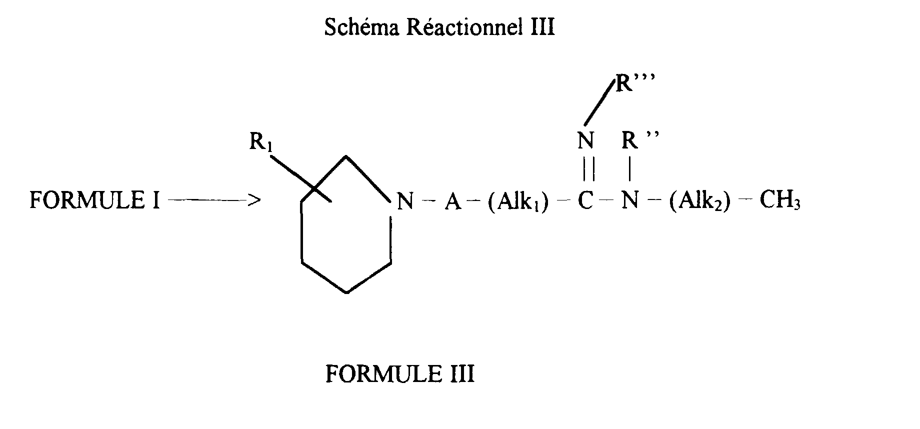
( C) pour ceux des composés selon la Formule A dans laquelle D est -CH₂-N-(R")-C(=NR"')-,
R"' est l'hydrogène, le fragment A est -CH₂- ou -CH(CH₃)-, E est une liaison directes y est égal à 0 et (Alk₁), (Alk₂), R1, R" et x sont tels que définis en premier lieu dans la Formule A, en effectuant la réaction décrite dans le Schéma Réactionnel IV en
(1) réalisant une réaction Pinner selon laquelle l'alkylnitrile 1, dans lequel Alk₂ a la même définition que celle désirée dans le produit final, est converti en ester amidate 3 et
(2) réalisant une réaction d'amidation entre ester amidate 3 et l'Intermédiaire II pourproduire le-composé de la Formule IV: 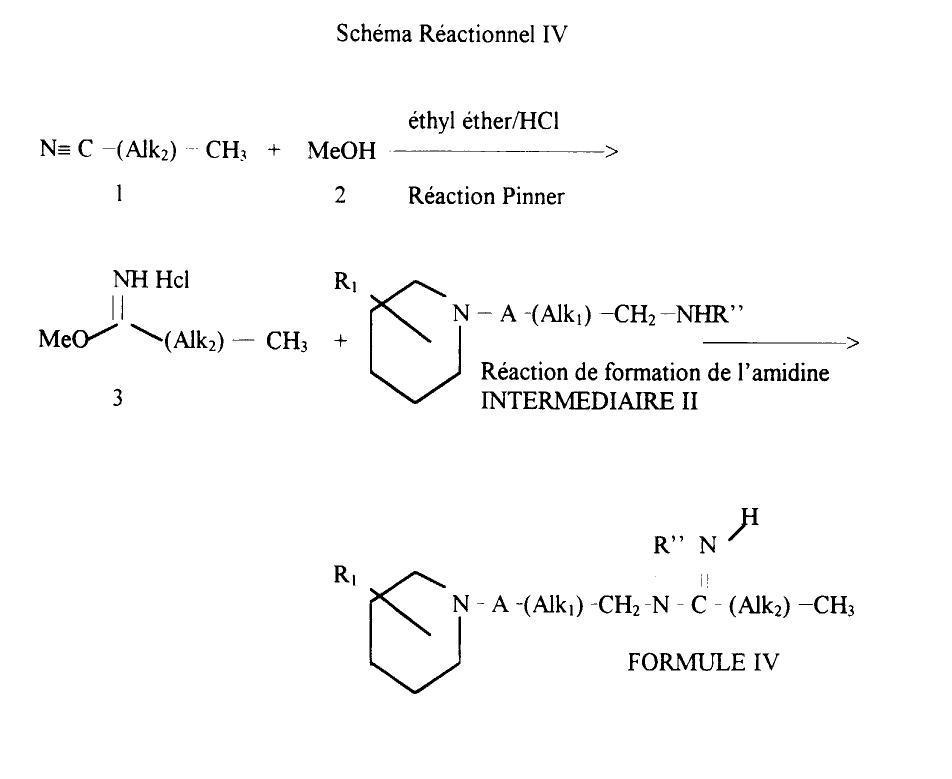 selon lequel on obtient l'Intermédiaire II, dans lequel R" est l'hydrogène (Intermédiaire IIA), en réalisant une réduction de l'alkylcyano pipéridine IA, dans laquelle A et Alk₁ ont les mêmes définitions que celles désirées dans le produit final, avec un agent réducteur, tel que l'hydrure de lithium aluminium: 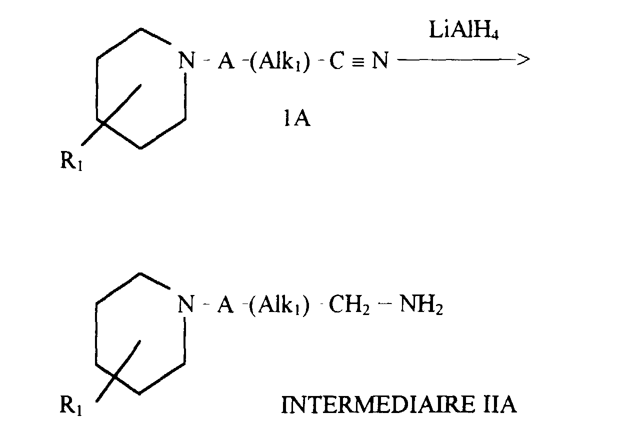 et selon lequel on obtient l'Intermédiaire II, dans lequel R" est un alkyle inférieur en C₁-C₄ (Intermédiaire IIB), de la manière suivante: 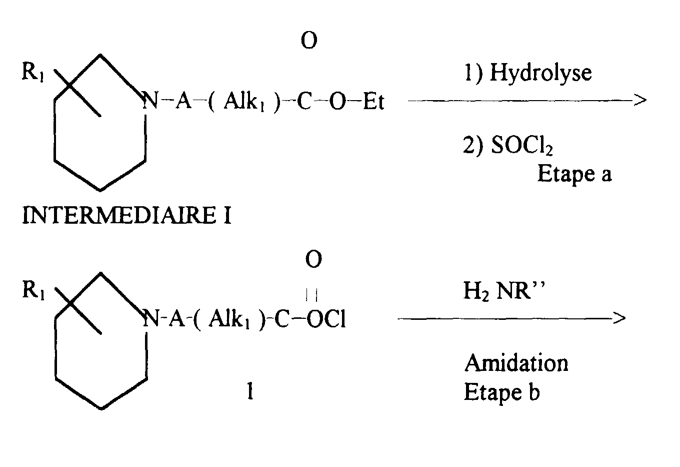 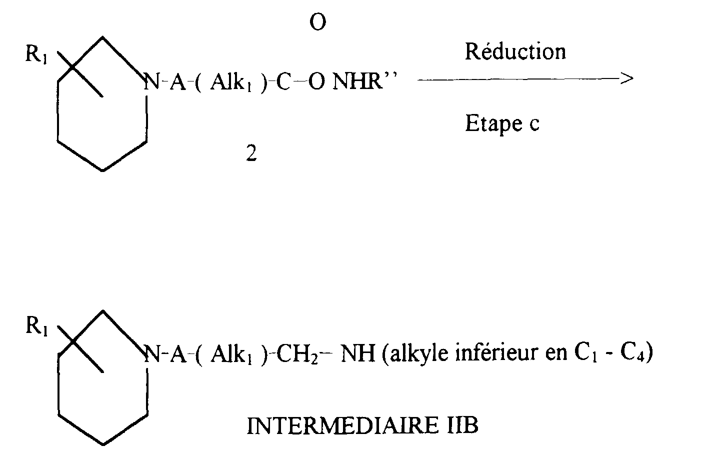
(D) pour ceux des composés selon la Formule A dans laquelle D est -CH₂-N(R")-C(O)-, E est une liaison directe, y est égal à 0 et les autres substituants sont tels que définis en premier lieu dans la Formule A, en effectuant la réaction décrite dans le schéma réactionnel V, en réalisant une hydrolyse acide de l'amidine de formule IV pour produire l'amide de Formule V: 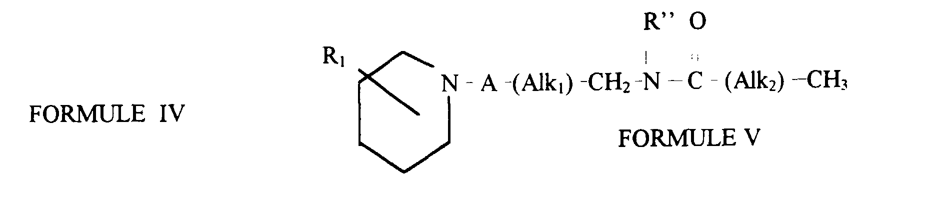
(E) pour ceux des composés selon la Formule A dans laquelle D est -CH₂-N(R")-C(=NR"')-, E est une liaison directe, y est égal à 0 et les autres substituants sont tels que définis en premier lieu dans la Formule A, en effectuant la réaction décrite dans le Schéma Réactionnel VI en (1) réalisant une alkylation de l'amide de Formule V avec un agent d'alkylation, tel que le tétrafluoroborate de triethyluxonium et (2) en réalisant une réaction de déplacement entre l'amide alkylé et un excès de l'amine désirée pour produire la Formule VI: 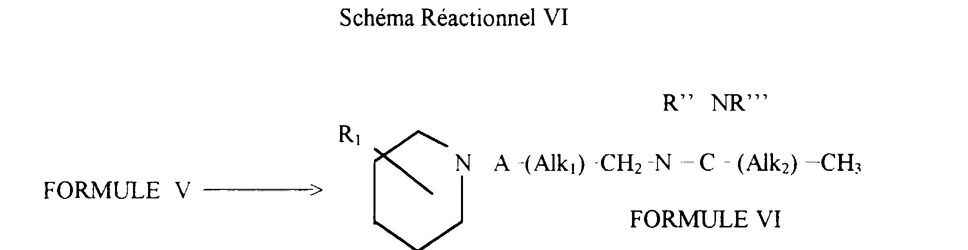
(F) pour ceux des composés selon la Formule A dans laquelle E est -C(=NR"')- N(R")- et les autres substituants sont tels que définis en premier lieu dans la Formule (A), en effectuant la réaction décrite dans le Schéma réactionnel X en
(1) réalisant une alkylation de l'amide de Formule IX avec un agent d'alkylation, tel que le tétrafluoroborate de triéthyloxonium et
(2) en réalisant une réaction de déplacement entre l'amide alkylé et un excès d'amine substituée H₂NR"', dans laquelle R"' a la même définition que dans le produit final désiré, pour produire le composé de la Formule X:

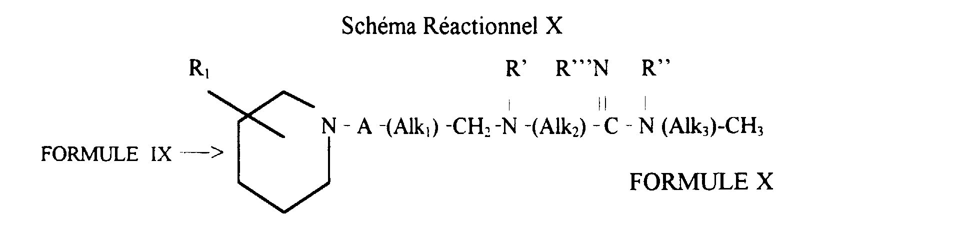

2. Procédé de préparation d'un composé selon la revendication 1, caractérisé en ce qu'il s'agit de la N-(3- pipéridinopropyl)-4-méthylvalérylamidine, et ses sels acceptables du point de vue pharmaceutique.

3. Procédé de préparation d'un composé selon la revendication 1, caractérisé en ce qu'il s'agit de la N-(4-pipéridinobutyl)-4-méthylvalérylamidine, et ses sels acceptables du point de vue pharmaceutique.

4. Procédé de préparation d'un composé selon la revendication 1, caractérisé en ce qu'il s'agit de la pipéridine octylamidine, et ses sels acceptables du point de vue pharmaceutique.

5. Procédé de préparation d'un composé selon la revendication 1, caractérisé en ce qu'il s'agit de la N-(1-trifluorométhyl-undécane)-pipéridine.

## Revendications (Revendications pour l'(les) Etat(s) contractant(s) suivant(s): GR)

1. Composés selon la formule (A): 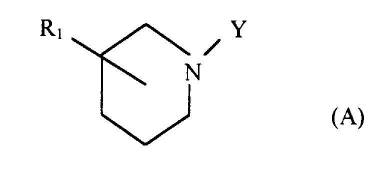 et ses sels acceptables du point de vue pharmaceutique, dans laquelle
Y représente -A-(Alk₁)ₓ-D-(Alk₂)-E-(Alk₃)_{y}-CH₃, dans lequel le fragment
A représente -CH₂ - ou 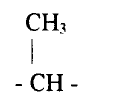 D et E représentent chacun indépendamment 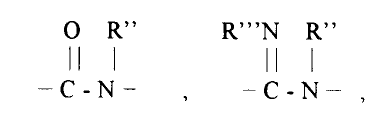 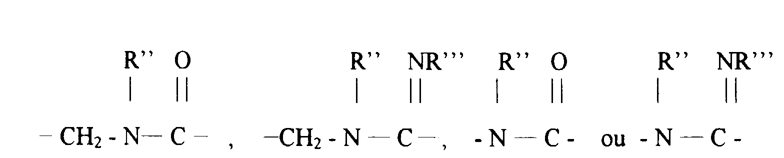 ou une liaison directe, à la condition que lorsque D représente un fragment choisi dans le groupe 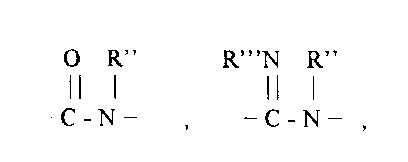 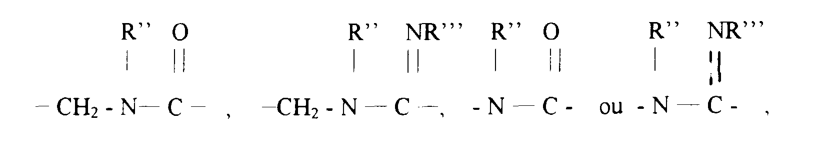 E ne puisse pas représenter un fragment du même groupe, et que D et E ne puissent pas être tous deux des liaisons directes;
(Alk₁), (Alk₂) et (Alk₃) représentent chacun indépendamment un fragment alkylène à chaîne linéaire contenant jusqu'à 5 atomes de carbone, éventuellement substitué par jusqu'à 3 groupes méthyle, ou
(Alk₁), (Alk₂) et (Alk₃) représentent chacun indépendamment un fragment alcénylène à chaîne linéaire contenant de 2 à 6 atomes de carbone, le fragment alcénylène à chaîne linéaire comportant de 1 à 2 double liaisons, et étant éventuellement substitué par jusqu'à 3 groupes méthyle;
R₁ représente l'hydrogène, un groupe hydroxy ou alkyle inférieur en C₁₋₄ ;
R', R", R"' représentent chacun indépendamment l'hydrogène ou un groupe alkyle inférieur en C₁₋₄ ;
et x et y représentent chacun indépendamment 0 ou 1; la condition que quand A est -CH₂- ou -CH(CH₃)- et l'un de D our E est une liaison directe, l'autre de D ou E n'est pas -CH₂-N(R')-, -N(R')- ou -C(O)N(R")- excepté quand le composé de formula (A) est
le N-(3-méthylbutyl)1-pipéridine propanamide,
le N-butyl-N-méthyl-1-pipéridinebutyramide ou
le 4-(3-méthylbutylamino)-1-pipéridinobutane et ses sels acceptables du point de vue pharmaceutique.

2. Composé selon la revendication 1, caractérisé en ce qu'il s'agit de la N-(3- pipéridinopropyl)-4-méthylvalérylamidine, et ses sels acceptables du point de vue pharmaceutique.

3. Composé selon la revendication 1, caractérisé en ce qu'il s'agit de la N-(4-pipéridinobutyl)- 4-méthylvalérylamidine, et ses sels acceptables du point de vue pharmaceutique.

4. Composé selon la revendication 1, caractérisé en ce qu'il s'agit de la pipéridine octylamidine, et ses sels acceptables du point de vue pharmaceutique.

5. Composé selon la revendication 1, caractérisé en ce qu'il s'agit de la N-(1-trifluorométhyl-undécane)-pipéridine.

6. Procédé de préparation d'un composé selon l'une des revendications 1 à 5, caractérisé en ce qu'il comprend une des étapes de procédé suivante (A) à (F):
(A) pour ceux des composés de formule A dans laquelle D est - C (O) - N(R") - , A est -CH₂ - ou - CH (CH₃)-, E est une liaison directe, y est égal à 0 et (Alk₁), (Alk₂), R" et x sont tels que définis en premier lieu dans la Formule A, en effectuant la réaction décrite dans le schéma réactionnel I en (1) réalisant la N-alkylation de la pipéridine 2 par un ester bromoalkylique 1, pour produire l'Intermédiaire I et (2) en réalisant une réaction d'amidation entre l'Intermédiaire I et une amine substituée 3 pour produire la Formule I: 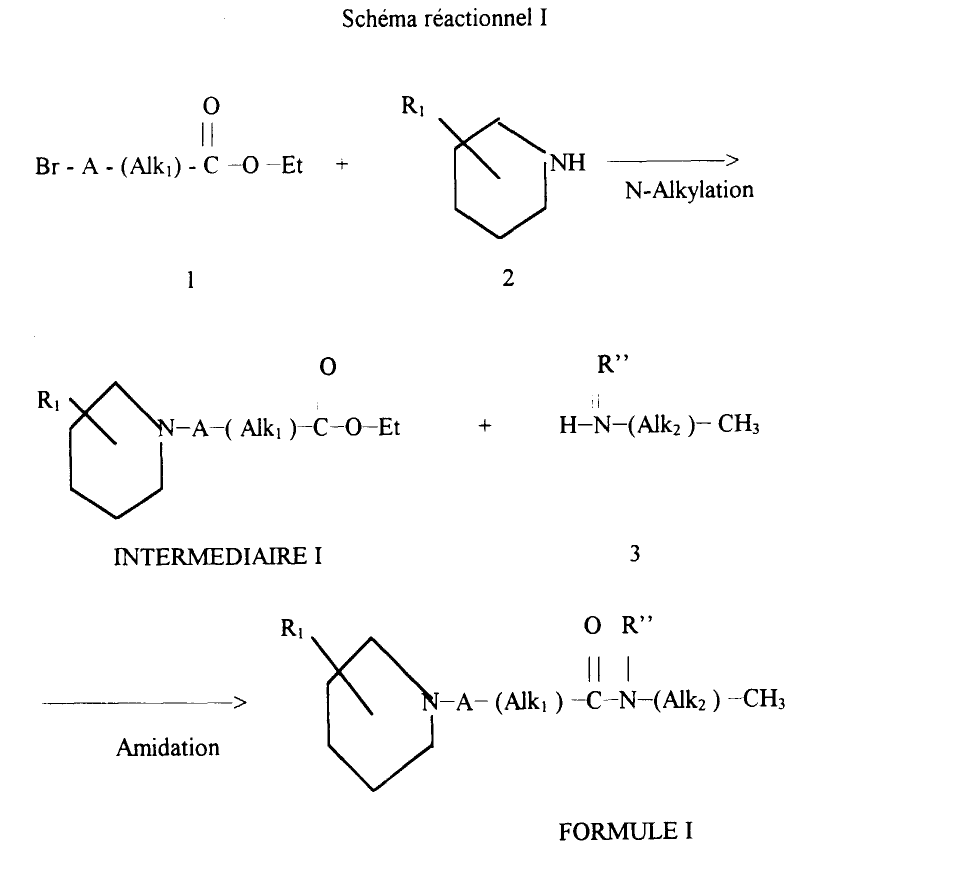
(B) pour ceux des composés selon la Formule A dans laquelle D est -C(=NR"')-N(R")-, E est une liaison directe et (Alk₃) est un fragment alkylène à 0 atomes de carbone et les autres substituants sont tels que définis en premier lieu dans la Formule A, en effectuant la réaction décrite dans le schéma réactionnel III en (1) réalisant une alkylation de l'amide de Formule I avec un agent d'alkylation, tel que le tétrafluoroborate de triéthyloxonium et (2) en réalisant une réaction de déplacement entre l'amide alkylé et une quantité en excès de l'amine désirée pour produire le composé de la Formule III: 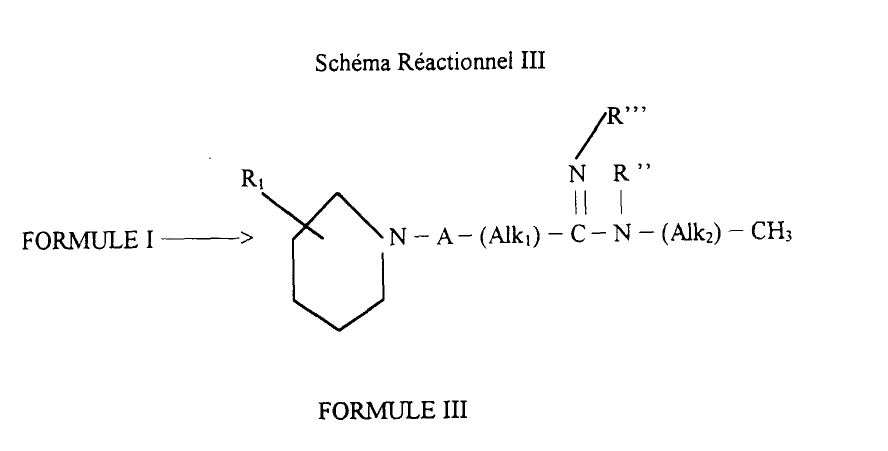
( C) pour ceux des composés selon la Formule A dans laquelle D est -CH₂-N-(R")-C(=NR"')-,
R"' est l'hydrogène, le fragment A est -CH₂- ou -CH(CH₃)-, E est une liaison directe, y est égal à 0 et (Alk₁), (Alk₂), R1, R" et x sont tels que définis en premier lieu dans la Formule A, en effectuant la réaction décrite dans le Schéma Réactionnel IV en
(1) réalisant une réaction Pinner selon laquelle l'alkylnitrile 1, dans lequel Alk₂ a la même définition que celle désirée dans le produit final, est converti en ester amidate 3 et
(2) réalisant une réaction d'amidation entre ester amidate 3 et l'Intermédiaire II pour produire le composé de la Formule IV: 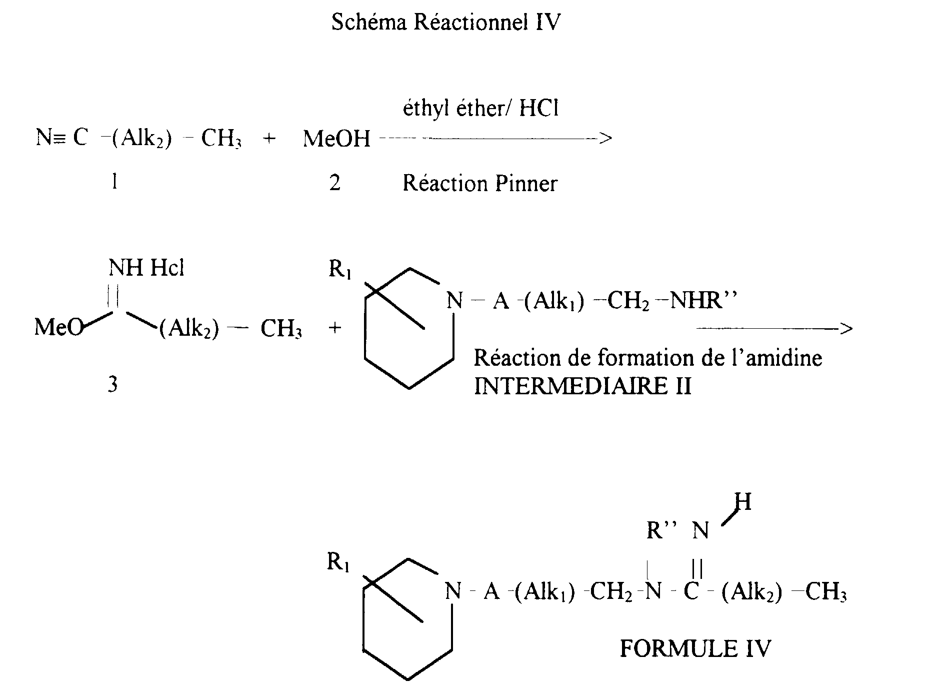 selon lequel on obtient l'Intermédiaire II, dans lequel R" est l'hydrogène (Intermédiaire IIA), en réalisant une réduction de l'alkylcyano pipéridine IA, dans laquelle A et Alk₁ ont les mêmes définitions que celles désirées dans le produit final, avec un agent réducteur, tel que l'hydrure de lithium aluminium: 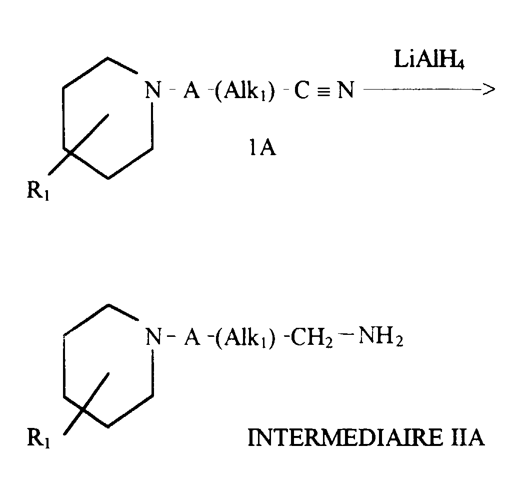 et selon lequel on obtient l'Intermédiaire II, dans lequel R" est un alkyle inférieur en C₁-C₄ (Intermédiaire IIB), de la manière suivante: 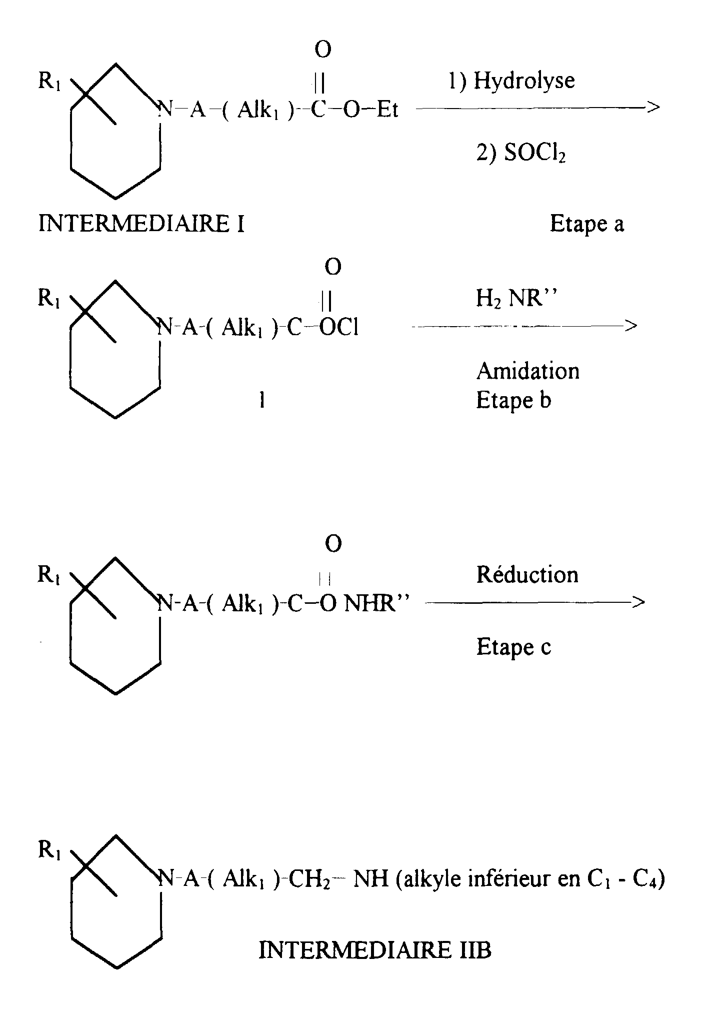
(D) pour ceux des composés selon la Formule A dans laquelle D est -CH₂-N(R")-C(O)-, E est une liaison directe, y est égal à 0 et les autres substituants sont tels que définis en premier lieu dans la Formule A, en effectuant la réaction décrite dans le schéma réactionnel V en réalisant une hydrolyse acide de l'amidine de Formule IV pour produire l'amide de Formule V: 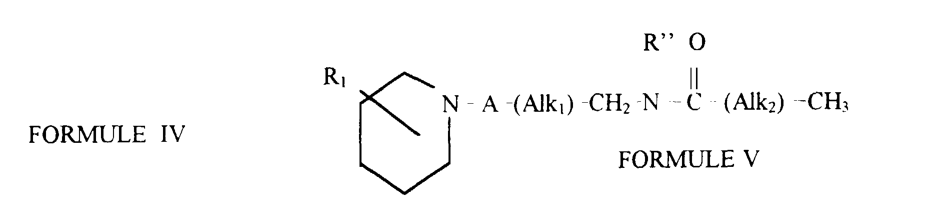
(E) pour ceux des composés selon la Formule A dans laquelle D est -CH₂-N(R")-C(=NR"')-, E est une liaison directe, y est égal à 0 et les autres substituants sont tels que définis en premier lieu dans la Formule A, en effectuant la réaction décrite dans le Schéma Réactionnel VI en (1) réalisant une alkylation de l'amide de Formule V avec un agent d'alkylation, tel que le tétrafluoroborate de triéthyloxonium et (2) en réalisant une réaction de déplacement entre l'amide alkylé et un excès de l'amine désirée pour produire le composé de la Formule VI: 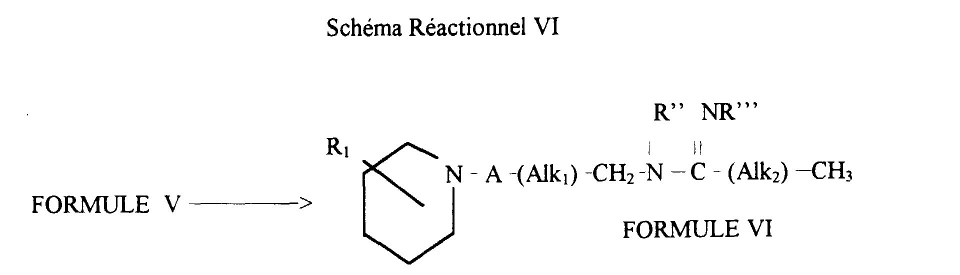
(F) pour ceux des composés selon la Formule A dans laquelle E est -C(=NR"')- N(R")-et les autres substituants sont tels que définis en premier lieu dans la Formule (A), en effectuant la réaction décrite dans le Schéma réactionnel X en
(1) réalisant une alkylation de l'amide de Formule IX avec un agent d'alkylation, tel que le tétrafluoroborate de triéthyloxonium et
(2) en réalisant une réaction de déplacement entre l'amide alkylé et un excès d'amine substituée H2NR"', dans laquelle R"' a la même signification que dans le produit final désiré, pour produire la Formule X:

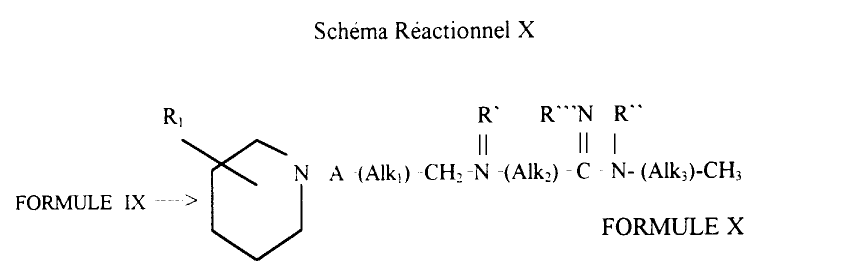

7. Utilisation d'un composé selon l'une des revendications 1 à 5 pour la préparation d'une composition pharmaceutique pour l'abaissement du cholestérol dans le plasma chez les mammifères.

8. Utilisation d'un composé selon l'une des revendications 1 à 5 pour la préparation d'une composition pharmaceutique pour le traitement d'infections fongiques chez les animaux.
